(19) Europäisches Patentamt
European Patent Office
Office européen des brevets

(11) EP 0 850 950 A1

(12) **EUROPEAN PATENT APPLICATION**
published in accordance with Art. 158(3) EPC

(43) Date of publication:
**01.07.1998 Bulletin 1998/27**

(21) Application number: **96929576.5**

(22) Date of filing: **10.09.1996**

(51) Int. Cl.[6]: **C07K 5/087**, C07K 5/107, C07K 5/023, A61K 38/06, A61K 38/07

(86) International application number:
**PCT/JP96/02573**

(87) International publication number:
**WO 97/10262 (20.03.1997 Gazette 1997/13)**

(84) Designated Contracting States:
**AT BE CH DE DK ES FI FR GB GR IE IT LI LU NL PT SE**

(30) Priority: **11.09.1995 JP 232162/95**
**22.04.1996 JP 100081/96**

(71) Applicants:
- **DAIICHI PHARMACEUTICAL CO., LTD.**
**Chuo-ku, Tokyo 103 (JP)**
- **Fuji Chemical Industries, Ltd.**
**Toyama 933 (JP)**

(72) Inventors:
- **SAKURADA, Shinobu**
**Miyagi 981-32 (JP)**
- **OKAYAMA, Toru**
**Fuji Chemical Industries, Ltd.**
**Toyama 933 (JP)**
- **NUKUI, Eriko**
**Fuji Chemical Industries, Ltd.**
**Toyama 933 (JP)**
- **HONGO, Kazuya**
**Fuji Chemical Industries, Ltd.**
**Toyama 933 (JP)**
- **OGAWA, Tadashi**
**Fuji Chemical Industries, Ltd.**
**Toyama 933 (JP)**
- **HONGO, Tomoko**
**Fuji Chemical Industries, Ltd.**
**Toyama 933 (JP)**
- **TAKESHIMA, Satoko**
**Fuji Chemical Industries, Ltd.**
**Toyama 933 (JP)**
- **TAKE, Nobuhiro**
**Fuji Chemical Industries, Ltd.**
**Toyama 933 (JP)**
- **NAKANO, Masaharu**
**Fuji Chemical Industries, Ltd.**
**Toyama 933 (JP)**

(74) Representative:
**Godemeyer, Thomas, Dr.**
**Hauptstrasse 58**
**51491 Overath (DE)**

(54) **PEPTIDE DERIVATIVES**

(57) A compound represented by the following formula or a salt thereof:

$$HN{=}C(R^1)\text{-}X\text{-}Y\text{-}NH\text{-}CH(R^2)\text{-}CO\text{-}Q$$

wherein $R^1$ represents an amino group or other.; $R^2$ represents a substituted benzyl group or other.; X represents a Pro residue, a substituted tyrosine residue or other.; Y represents an alanine residue or other.; and Q represents N-methyl-β-alanine or other. The compounds have analgesic effect, and can be used for the treatment of cancer pain etc.

EP 0 850 950 A1

## Description

### Technical Field

The present invention relates to peptide derivatives exhibiting pharmaceutical activities such as analgesic activity through action on opioid receptors and the like.

### Background Art

The existence of opioid receptors to which opioids such as morphine bind was verified in the early 1970's. At present, opioid receptors are mainly classified into three types, i.e., $\mu$, $\delta$ and $\kappa$. Morphine mostly acts on the $\mu$ acceptor as an agonist and exhibits pharmaceutical activities such as analgesic activity, enterokinetic inhibition, and respiratory inhibition.

Since 1975, several endogenous morphine-like substances that bind to the opioid receptors have been successively discovered. All of these substances found to date are peptide compounds and are collectively referred to as opioid peptides. The pharmaceutical activities of the opioid peptides are believed to be basically the same as those of morphine. They are expected to be potentially safer drugs than morphine since they are substances naturally exist in living bodies. However, natural opioid peptides have problems from the pharmacokinetic standpoint, and they have not been used as clinical medicaments.

In the 1980's, Delmorphine that contains D-alanine was isolated from cutises of frogs. It was found that Delmorfine has about 1000-fold higher analgesic effect than morphine at intraventricular administration and is relatively stable in living bodies. Since then, synthetic opioid peptides containing D-amino acids have been prepared. In particular, synthetic opioid peptides with high $\kappa$ acceptor selectivity are seen as hopeful non-narcotic analgesics and clinical trials have begun. However, the probability of their success as clinical medicaments is doubtful from the viewpoints of efficacy, possible side effects due to properties as $\kappa$ agonists, and commercial practicability.

Furthermore, it is impossible to use these synthesized opioid peptides as orally available medicaments, and accordingly, they can not be substitutive drugs for, for example, MS contin, which is an orally available controlled release preparation comprising morphine sulfate that has been widely used recently as a medicament for treatment of cancerous pain. On the other hand, daily dose of MS contin may occasionally be increased up to gram order, which sometimes leads to difficulty in oral administration. In addition, in some cases, its administration cannot be continued because of side effects such as pruritus due to its activity on the release of histamine. Therefore, substitutive medicaments are desired which have higher safety and efficacy than morphine.

### Disclosure of the Invention

In order to achieve the aforementioned objects, the inventors of the present invention conducted various studies aimed at providing opioid peptide derivatives having excellent analgesic activity and oral absorbability. As a result, they found that oligopeptide derivatives and their salts having a basic structure of L-Tyr-(L or D)-Arg-Phe and an amidino group at their N-terminals have the desired properties, and filed patent applications pertaining to these peptide derivatives (Japanese Patent Applications Nos. (Hei)6-40989/1994 and (Hei)7-49894/1995). The inventors conducted further studies and found that novel peptide compounds obtained by various modifications to the above basic structure also have the desired properties. The present invention was achieved on the basis of these findings.

The peptide derivatives of the present invention are represented by the following Formula I: $HN{=}C(R^1)$-X-Y-NH-$CH(R^2)$-CO-Q.

According to the present invention, a medicament consisting of said compound or a salt thereof; and a analgesic pharmaceutical composition comprising said compound or a salt thereof as an active ingredient are provided. A use of said compound or a salt thereof for manufacture of the aforementioned pharmaceutical composition; and method for preventive and/or therapeutic treatment of pain comprising a step of administering to a mammal an effective amount of said compound or a salt thereof are also provided according to the present invention.

### Best Mode for Carrying Out the Invention

In the above Formula I, $R^1$ represents an amino group which may optionally be substituted or a $C_{1-6}$ (1 to 6 carbon atoms) alkyl group. Where the amino group is substituted, the number of the substituent may be one or two, and where two susbsituents exist, they may be the same or different. As the substituent, for example, a $C_{1-6}$ alkyl group, hydroxyl group, a substituted or unsubstituted amino group may be used. The $C_{1-6}$ alkyl referred to in the specification may be linear or branched. For example, methyl group, ethyl group, n-propyl group, isopropyl group, n-butyl group, sec-butyl group, tert-butyl group, n-pentyl group, isopentyl group, neopentyl group, n-hexyl group and the like may be used. $R^1$ is

preferably an unsubstituted amino group or methyl group.

$R^2$ represents a $C_{1-6}$ alkyl group substituted with an aryl group which may optionally be substituted or with a $C_{3-6}$ cycloalkyl group which may optionally be substituted. Although a position of the substitution on the $C_{1-6}$ alkyl group by the aryl group or the cycloalkyl group is not particularly limited, the substitution may preferably be at the terminal of the $C_{1-6}$ alkyl group. Where the aryl group or the cycloalkyl group is substituted, they may have one or more substituents at any substitutable positions. Examples of the substituents on the aryl group or the cycloalkyl group include, for example, hydroxyl group, an amino group which may optionally be substituted, a guanidino group which may optionally be substituted, a $C_{1-6}$ alkyl group, a $C_{1-6}$ alkyloxy (alkoxyl) group, a halogen atom (the term "halogen atom" herein used means any one of fluorine atom, chlorine atom, bromine atom, and iodine atom). For example, p-hydroxybenzyl group, phenethyl group, cyclohexylmethyl group, p-chlorobenzyl group, p-fluorobenzyl group are preferred as $R^2$. Although the absolute configuration of the carbon atom bound to $R^2$ is not particularly limited, compounds where the absolute configuration is S-configuration are preferred according to the present invention.

X represents a proline (Pro) residue which may optionally be substituted. Although the proline residue may be an L-proline residue or a D-proline residue, a D-proline residue is preferred. Where the proline residue is substituted, it may have one or more substituents at any positions. Where two or more substituents exist, the substituents may be the same or different. As the substituted proline residue, 4-hydroxyproline, for example, may be preferably used.

X also represents a group defined by the following formula:

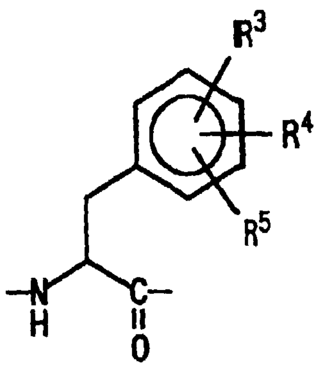

In the above formula, $R^3$, $R^4$ and $R^5$ independently represent hydrogen atom, hydroxyl group, a $C_{1-6}$ alkyl group, a $C_{1-6}$ alkoxyl group, a halogen atom, or $-OSO_3H$. $R^3$, $R^4$ and $R^5$ may be the same or different. For example, compounds wherein all of $R^3$, $R^4$ and $R^5$ are hydrogen atoms; compounds wherein $R^3$ is 4-hydroxyl group, and $R^4$ and $R^5$ are methyl groups at 2-position and 6-position, respectively; compounds wherein $R^3$ is 4-hydroxyl group, and $R^4$ and $R^5$ are hydrogen atoms; compounds wherein $R^3$ is 4-methoxy group, and $R^4$ and $R^5$ are hydrogen atoms; compounds wherein $R^3$ and $R^4$ are hydroxyl groups at 3-position and 4-position, respectively, and $R^5$ is hydrogen atom; and compounds wherein $R^3$ is $-OSO_3H$, and $R^4$ and $R^5$ are hydrogen atoms and the like are preferred. Compounds wherein X is a tyrosine (Tyr) residue, particularly an L-Tyr residue, are most preferred embodiments of the present invention.

Y represents D-proline (Pro) residue, L-proline (Pro) residue, or a group defined by the formula set out below.

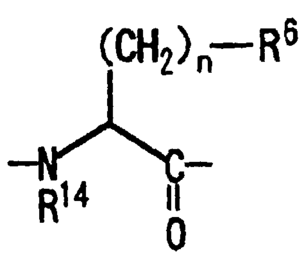

In the above formula, $R^6$ represents hydrogen atom, an amino group which may optionally be substituted, a guanidino group ($-NH-C(NH_2)=NH$) which may optionally be substituted, a mercapto group (-SH) which may optionally be substituted, a phenyl group which may optionally be substituted, a ureido group ($-NH-CO-NH_2$) which may optionally be substituted, a carbamoyl group ($-CO-NH_2$) which may optionally be substituted, a thioureido group ($-NH-CS-NH_2$)

which may optionally be substituted, a $C_{1-6}$ alkylsulphinyl group (-SO-$C_{1-6}$ alkyl), a $C_{1-6}$ alkylsulfonyl group (-$SO_2$-$C_{1-6}$ alkyl), a $C_{1-6}$ acyloxy group, carboxyl group, or a $C_{1-6}$ alkoxycarbonyl group, n represents an integer of from 1 to 4, and $R^{14}$ represents hydrogen atom or a $C_{1-6}$ alkyl group.

Where $R^6$ represents hydrogen atom, the above formula preferably represents D-alanine (Ala) residue, L-alanine residue, D-norvaline (Nva) residue, L-norvaline (Nva) residue or the like. Where the amino group is substituted, the number of the substituent may be one or two. As the substituent on the amino group, for example, imidazolynyl group, a $C_{1-6}$ alkyl group, an imino($C_{1-6}$ alkyl) group such as 1-iminoethyl group, a $C_{1-6}$ alkanoyl group such as acetyl group, or a halogenated $C_{1-6}$ alkanoyl group such as trifluoroacetyl group may be used. An amino group having one substituent such as methyl group, ethyl group, isopropyl group, 1-iminoethyl group, or acetyl group may preferably be used.

Where the guanidino group is substituted, the nitrogen atom constituting the guanidino group may be substituted with one or more substituents, and where two or more substituents exist, the substituents may be the same or different. For example, a $C_{1-6}$ alkyl group, hydroxyl group, a halogen atom, nitro group, a $C_{1-6}$ alkanoyl group such as acetyl group, a halogenated $C_{1-6}$ alkanoyl group such as trifluoroacetyl group or the like may be used, and methyl group may preferably be used.

Where $R^6$ is a substituted mercapto group, the substituent may be a $C_{1-6}$ alkyl group, preferably methyl group and the like. Where $R^6$ is a substituted phenyl group, it may have one or more substituents at any positions on the phenyl ring. As the substituent, for example, the optionally substituted amino group or the optionally substituted guanidino group explained above may be used. For example, p-aminophenyl group, p-guanidinophenyl group and the like may preferably be used.

Where $R^6$ is a substituted ureido group, a substituted carbamoyl group, or a substituted thioureido group, as the substituent, for example, a $C_{1-6}$ alkyl group, a $C_{1-6}$ alkanoyl group such as acetyl group, a halogenated $C_{1-6}$ alkanoyl group such as trifluoroacetyl group and the like may be used. Where $R^6$ is a $C_{1-6}$ alkylsulphinyl group or a $C_{1-6}$ alkylsulphonyl group, methyl group, for example, may preferably be used as the $C_{1-6}$ alkyl group that substitutes on the sulphinyl group or the sulfonyl group.

As the $C_{1-6}$ acyloxy group represented by $R^6$, a $C_{1-6}$ alkanoyloxy group such as acetyloxy group or a halogenated $C_{1-6}$ alkanoyloxy group such as trifluoroacetyloxy group may be used. Alternatively, an aryl-substituted carbonyloxy group, e.g., benzoyloxy group, which may optionally have one or more substituents on the ring may be used as $R^6$. As the substituent on the phenyl ring of the benzoyl group, for example, a $C_{1-6}$ alkyl group, a halogen atom, or the optionally substituted amino group explained above may be used. Where $R^6$ represents carboxyl group, the above formula preferably represents D-glutaminic acid (Glu) residue or L-glutaminic acid residue. Examples of $R^6$ as being the $C_{1-6}$ alkoxy carbonyl group include, for example, methoxycarbonyl group, ethoxycarbonyl group and the like.

The compounds where $R^{14}$ represents hydrogen atom are preferred compounds of the present invention. Where $R^6$ is a substituted or unsubstituted guanidino group, preferably a non-substituted guanidino group and n is 3, $R^{14}$ is preferably a $C_{1-6}$ alkyl group such as methyl group. Although the configuration of the $\alpha$-carbon atom of the residue represented by Y is not particularly limited, a D-amino acid residue may be preferred from the viewpoint of stability in living bodies. For example, the compounds comprising a residue selected from D-alanine residue, D-glutaminic acid residue, D-norvaline residue, D-proline residue, N-methyl-D-arginine residue, citrulline residue, and D-methionine sulfoxide residue are preferred compounds of the present invention.

Q represents -$OR^7$, -N($R^8$)($R^9$) or -$NR^{10}$-C($R^{11}$)($R^{12}$)($R^{13}$). $R^7$ represents hydrogen atom or a $C_{1-6}$ alkyl group, and $R^8$ represents hydrogen atom or a $C_{1-6}$ alkyl group. $R^9$ represents a $C_{1-6}$ hydroxyalkyl group or a $C_{1-6}$ alkyl group substituted with sulfonic acid. Although the hydroxyl group and the sulfonic acid group may exist at any position of the alkyl group, an alkyl group substituted at the terminal is preferred. $R^8$ and $R^9$ may combine to form a 5- or 6-membered nitrogen-containing saturated heterocyclic group together with the nitrogen atom to which $R^8$ and $R^9$ bind. The heterocyclic group may contain two or more nitrogen atoms. For example, 1-piperadinyl group, 1-pyrrolidinyl group, 1-piperidinyl group or the like may be used as -$NR^8R^9$.

Where X represents $NR^{10}$-C($R^{11}$)($R^{12}$)($R^{13}$), $R^{10}$ represents hydrogen atom, a $C_{1-6}$ alkyl group, or a $C_{1-6}$ alkyl group substituted with an aryl group such as phenyl group (an aralkyl group). An example of the aralkyl group includes benzyl group. $R^{10}$ is preferably methyl group. $R^{11}$ represents hydrogen atom; carboxyl group; a carbonyl group substituted with a $C_{1-6}$ alkoxyl group such as methoxycarbonyl and ethoxycarbonyl; a substituted or unsubstituted carbamoyl group; a $C_{1-6}$ alkyl group substituted with carboxyl group; a $C_{1-6}$ alkyl group substituted with a substituted or unsubstituted carbamoyl group; or a $C_{1-6}$ alkyl group having a carbonyl group substituted with a $C_{1-6}$ alkoxyl group. For example, $R^{11}$ is preferably -$CH_2$-COOH.

$R^{12}$ represents hydrogen atom; a $C_{1-6}$ alkyl group; an amino-$C_{1-6}$ alkyl group; a $C_{1-6}$ alkyl group substituted with an amidino group; a $C_{1-6}$ alkyl group substituted with a guanidino group; a hydroxy-$C_{1-6}$ alkyl group; a $C_{1-6}$ alkyl group substituted with a carboxyl group; or a $C_{1-6}$ alkyl group substituted with a substituted or unsubstituted carbamoyl. Alternatively, $R^{10}$ and $R^{12}$ may combine to form a 5- or 6-membered nitrogen-containing saturated heterocyclic group having carboxyl group on the ring together with the nitrogen atom to which $R^{10}$ binds. Examples of the heterocyclic group include, for example, 2-carboxy-1-pyrrolidinyl group (-Pro-OH) and 3-carboxy-1-piperidinyl group. $R^{13}$ represents hydro-

gen atom or a $C_{1-6}$ alkyl group. For example, combinations in which $R^{11}$ is carboxymethyl group or carbamoylmethyl group and $R^{12}$ and $R^{13}$ are hydrogen atoms, and the aforementioned combinations in which $R^{10}$ is methyl group (i.e., Q represents $-NCH_3-CH_2-CH_2-COOH$) are preferred.

Compounds of the above Formula I where $R^2$ is unsubstituted benzyl group, X is L-tyrosine (L-Tyr) residue (i.e., $R^3$ is p-hydroxyl group, $R^4$ and $R^5$ are hydrogen atoms, and the configuration corresponds to that of L-amino acid), and Y is L- or D-arginine (Arg) residue (i.e., $R^6$ is non-substituted guanidino group and n is 3) are excluded from the scope of the present invention.

Asymmetric carbons present in the compounds of the present invention represented by Formula I may be in S- or R-configuration, other than those defined above. Where a sulfur atom substituted with an oxygen atom, e.g., sulfoxides, may have S- or R-configuration, the configuration may be either of these configurations. For example, where Y represents D-methionine sulfoxide residue, D-methionine-(RS)-sulfoxide residue, D-methionine-(R)-sulfoxide residue, and D-methionine-(S)-sulfoxide residue fall within the scope of the present invention.

Any optical isomers or racemates, diastereomers, and mixtures of such isomers fall within the scope of the compounds of the present invention represented by Formula I. Acid addition salts such as hydrochlorides, acetates, and p-toluenesulfonates, or base addition salts such as ammonium salts and organic amine salts fall within the scope of the compounds of the present invention. Furthermore, any hydrates and solvates of the free form or the salt form of the compounds also fall within the scope of the present invention. In addition to the compounds represented by the above formula, dimers or oligomers derived from the above compounds and cyclic compounds produced by binding of C-terminals and N-terminals of these compounds also fall within the scope of the compounds of the present invention.

The peptides of the present invention have higher analgesic activity than morphine. Since their analgesic activity is accompanied by relatively weaker effects on the release of histamine and heart rate depression than those of morphine, and since their degree of cross resistance between morphine is low, they can be expected to be suitable for the treatment of cancerous pain. Therefore, according to the present invention, there are provided a pharmaceutical composition comprising the above compound. Examples of the route of administration include, for example, intravenous administration, subcutaneous administration, and oral administration. Formulations for mucosal absorption including nasal absorption and formulations for endermic absorption are also expected to be useful. Dose of administration is not particularly limited. For example, a single dose of 0.1 to 10 mg for subcutaneous administration or a single dose of 1 to 100 mg for oral administration may be administered twice or three times a day.

The peptide derivatives of the present invention can be prepared by a solid phase method or a liquid phase method ordinarily used for peptide preparations. For example, peptide chains may first be prepared without amidino groups by a solid phase method, and amidino groups may be introduced into the amino group of N-terminal tyrosine to obtain desired peptide derivatives. Alternatively, amidino groups may be introduced beforehand and the C-terminal then be modified.

Various excellent agents are available as protective groups for amino groups and the like and as condensing agents and the like for condensation reactions. These can be suitably selected and used with reference to Examples set out below, or in view of, for example, Koichi Suzuki Ed., "Tanpakushitu Kohgaku-Kiso to Ohyo (Protein Engineering: Fundamentals and Applications)," Maruzen Co., Ltd. (1992) and publications cited therein; M. Bondanszky, et al., "Peptide Synthesis," John Wiley & Sons, N.Y., 1976; and J.M. Stewart and D.J. Young, "Solid Phase Peptide Synthesis," W.H. Freeman and Co., San Francisco, 1969. For the solid phase methods, various commercially available peptide synthesizers, for example, Model 430A (Perkin Elmer Japan, formerly Applied Biosystems), may conveniently be used. Resins, reagents and the like used in the preparations are easily obtainable as commercially available products and examples are indicated in Examples.

## Examples

The present invention will be more specifically explained by examples. However, the present invention is not limited to these examples. By referring to the examples, modifying or altering the methods of the examples, or appropriately selecting starting materials or reagents for reactions, desired peptide derivatives of the present invention that fall within the scope of Formula I can easily be prepared. In the examples, the meanings of amino acid groups are similar to those ordinarily used. Where an amino acid having D-form or L-form is referred to, the amino acid represents the L-amino acid unless specifically described as D-form. In addition, the following abbreviations will be used, and similar abbreviations not specifically defined below will also be used. The descriptions $H_2NC(NH)$-Phe-, Boc-Phe-, and BZA-Phe-, for example, mean that a nitrogen atom at the N-terminal of phenylalanine residue is substituted with $H_2NC(NH)$-, Boc, and BZA, respectively. Amino acids occasionally mean amino acid residues.

AGBA: 2-amino-4-guanidinobutyric acid,
AGHX: 2-amino-6-guanidinohexanoic acid,
AGPR: 2-amino-3-guanidinopropionic acid,

Alko resin: p-alkoxybenzyl alcohol resin [4-hydroxymethylphenoxymethyl-co-polystyrene, 1% divinylbenzene resin, J. Am. Chem. Soc., 95, 1328 (1974), Watanabe Chemical Industry],
AMSB or Met(O): 2-amino-4-methylsulphinylbutyric acid,
APBA or HomoPhe: 2-amino-4-phenylbutyric acid,
Boc: t-butoxycarbonyl group,
BPBA: 2-amino-3-phenylbutyric acid,
BZA: N,N'-bis(benzyloxycarbonyl)amidino group,
Cha: cyclohexylalanine,
Cit: citrulline [$NH_2$-CO-NH-$(CH_2)_3$-CH($NH_2$)-COOH],
DABA: 2,4-diaminobutyric acid,
DAHX or Lys: 2,6-diaminohexanoic acid,
DAPE or Orn: 2,5-diaminopentanoic acid,
DAPR: 2,3-diaminopropionic acid,
DCM: dichloromethane,
DIPEA: N,N-diisopropylethylamine,
DIPCI: N,N-diisopropylcarbodiimide,
DMAP: N,N-dimethylaminopyridine,
DMF: dimethylformamide,
DMSO: dimethylsulfoxide,
DMT: (2',6'-dimethyl)tyrosine,
DOPA: (3',4'-dihydroxy)phenylalanine,
EDC: 1-(3-dimethylaminopropyl)-3-ethyl-carbodiimide,
Fmoc: 9-fluorenylmethyloxycarbonyl,
Fmoc-NH-SAL resin: 4-(2',4'-dimethoxyphenyl-9-fluorenylmethoxycarbonylaminomethyl)phenoxy resin (Watanabe Chemical Industry),
H-β Ala-ol: $NH_2CH_2CH_2CH_2OH$,
HBTU: 2-(1H-benzotriazole-1-yl)-1,1,3,3-tetramethyluronium • hexafluorophosphate,
HOBt: 1-hydroxybenzotriazole,
Hyp: 4-hydroxyproline,
IEPE: 2-amino-5-(1-iminoethylamino)pentanoic acid,
IEPR: 2-amino-3-(1-iminoethyl)aminopropionic acid,
IPPE: 2-amino-5-isopropylaminopentanoic acid,
MeβAla or βMeAla: N-methyl-β-alanine, MeTyr: N-methyltyrosine,
MGPE or Arg($CH_3$): 2-amino-5-methylguanidinopentanoic acid,
(N-Me)Arg: N-methylarginine,
NMP: N-methylpyrrolidone,
Nva: norvaline,
OBzl: benzyloxy group,
Orn(Ac): $N^5$-acetylornithine,
Orn(Tfa): $N^5$-trifluoroacetylornithine,
OTce: trichloroethyl ester group,
PAPA: (4-amino)phenylalanine,
PGPA: p-guanidinophenylalanine,
Phe(p-Cl): (4-chloro)phenylalanine,
Phe(p-F): (4-fluoro)phenylalanine,
Pmc: 2,2,5,7,8-pentamethylchroman-6-sulfonyl,
PMPA: p-methoxyphenylalanine,
PyBrop: bromotris(pyrrolidino)phosphonium • hexafluorophosphate,
t-Bu: tertiary-butyl,
TEA: triethylamine,
TFA: trifluoroacetic acid,
Tos: p-toluenesulfonyl,
TosOH: p-toluenesulfonic acid,
Troc: 2,2,2-trichloroethoxycarbonyl group,
Tyr($SO_3H$) : tyrosine-O-sulfate,
WSCI: 1-ethyl-3-(3-dimethylaminopropyl)-carbodiimide,
Z: benzyloxycarbonyl group.

(A) Preparation of starting materials

(1) H-Tyr-D-Arg-Phe-βAla-OH

The above peptide was prepared by the solid phase method (Original Autoprogram for the Fmoc/NMP method) using a Model 430A peptide synthesizer (Applied Biosystems, ABI) as follows. Fmoc-βAla-Alko resin (0.25 mmol, 675 mg) was washed once with NMP and treated with NMP containing 20% piperidine for 4 minutes, and then with NMP containing 20% piperidine for 16 minutes. The resin was washed with NMP 5 times, and then allowed to react with Fmoc-Phe-OH for 61 minutes. Then, the resin was washed with NMP four times and recovered from the fourth rinse, and unreacted amino groups were allowed to react with acetic anhydride. The above-described first cycle was carried out in 120 minutes, and similar procedure was repeated using Fmoc-D-Arg(Pmc)-OH for the second cycle, and Fmoc-Tyr(t-Bu)-OH for the third cycle instead of Fmoc-Phe-OH. As side chain protective groups, Pmc was used for D-Arg and t-Bu for Tyr.

500 mg of the resin obtained in the above procedure was treated with stirring in a mixture of phenol (crystal, 0.75 g), ethanedithiol (0.25 ml), thioanisole (0.50 ml), water (0.50 ml), and TFA (10.0 ml) at room temperature for 3 hours to liberate peptides from the resin and simultaneously remove the protective groups. Then, the mixture was filtered using a 3 μm filter (ADVANTEC-Polyflon filter), cold diethyl ether (200 ml) was added to the filtrate, and the resulting precipitates were collected by filtration using a 3 μm filter (ADVANTEC-Polyflon filter). The precipitates on the filter were dissolved in 10 to 20 ml of 2N acetic acid and lyophilized to give crude peptide of the formula H-Tyr-D-Arg-Phe-βAla-OH.

(2) H-Tyr-D-Arg-Phe-$NHCH_2CH_2CONH_2$

The above peptide was obtained in the same manner as in the above (1) except that Fmoc-NH-SAL resin (0.25 mmol, 385 mg) was used at the beginning of the synthesis.

(3) H-Tyr-D-Arg-Phe-βMeAla-OH

The above peptide was prepared by a solid phase method according to the Fmoc/NMP method as follows. A glass filter was used for filtration. After Alko resin (0.500 g) had been swollen with DMF (6 ml), the resin was added with Fmoc-N-methyl-β-alanine (Fmoc-βMeAla-OH, 0.228 g) and pyridine (0.093 ml), shaken for 1 minute, then added with 2,6-dichlorobenzoyl chloride (0.147 g), and shaken for 24 hours. The resulting Fmoc-βMeAla-Alko resin was washed three times with 6 ml of DMF, then three times with 6 ml of methanol and further three times with 6 ml of DCM, and unreacted hydroxymethyl groups were benzoylated by adding benzoyl chloride (0.0891 ml) and pyridine (0.0847 ml) in DCM (6 ml) and shaking for 1 hour. The amino acid resin was successively washed three times with 6 ml of DCM, three times with 6 ml of DMF and three times with 6 ml of methanol, and dried in vacuum in a desiccator over potassium hydroxide.

The Fmoc-β-MeAla-Alko resin was treated three times with 12 ml of DMF, then three times with 12 ml of DMF containing 20% piperidine and further six times with 12 ml of DMF to remove the Fmoc group, and then added with Fmoc-Phe-OH (0.262 g), PyBrop (Watanabe Chemical Industry, 0.315 g), NMP (6 ml) and DIPEA (0.273 ml) and shaken for 24 hours to form Fmoc-Phe-βMeAla-Alko resin. After filtration and washing with 6 ml of NMP, unreacted amino groups were capped by treatment with DMF (6 ml) containing 1-acetylimidazole (0.248 g) and DIPEA (0.0784 ml) for 1 hour. The resulting resin was then washed with 6 ml of NMP.

The Fmoc group was removed from the Fmoc-Phe-β-MeAla-Alko resin in the same manner as described above, and the resultant was added with Fmoc-D-Arg(Pmc)-OH (0.557 g), HOBt (0.121 g), HBTU (0.299 g) and DIPEA (0.274 ml), and shaken for 1 hour to form Fmoc-D-Arg(Pmc)-Phe-β-MeAla-Alko resin. After filtration and washing, unreacted amino groups were capped in the same manner as described above.

The Fmoc group was removed from the Fmoc-D-Arg(Pmc)-Phe-βMeAla-Alko resin in the same manner as described above, and the resultant was added with Fmoc-Tyr(t-Bu)-OH (0.310 g), HOBt (0.103 g), HBTU (0.256 g) and DIPEA (0.235 ml), and shaken for 1 hour to form Fmoc-Tyr(t-Bu)-D-Arg(Pmc)-Phe-βMeAla-Alko resin. Alter filtration and washing, unreacted amino groups were capped in the same manner as described above. The peptide was liberated from the resin and the protective group was simultaneously removed in the same manner as in the above (1).

(4) H-Tyr-D-Arg-Phe-βEtAla-OH

The above peptide was prepared by a solid phase method according to the Fmoc/NMP method as follows. A glass filter was used for filtration. Alter Alko resin (1.000 g) had been swollen with NMP (12 ml), the resin was added with Fmoc-N-ethyl-β-alanine (Fmoc-βEtAla-OH, 0.475 g) and DMAP (0.017 g), shaken for 1 minute, then added with DIPCI (0.177 g), and shaken for 24 hours. The resulting Fmoc-βEtAla-Alko resin was washed three times with 12 ml of NMP, then three times with 12 ml of methanol, and further three times with 12 ml of DCM, and unreacted hydroxymethyl

groups were benzoylated by adding benzoyl chloride (0.178 ml) and pyridine (0.170 ml) in DCM (12 ml) and shaking 1 hour. The amino acid resin was successively washed three times with 12 ml of DCM, three times with 12 ml of DMF, and three times with 12 ml of methanol, and dried in vacuum in a desiccator over potassium hydroxide.

The Fmoc-βEtAla-Alko resin was treated three times with 20 ml of DMF, then three times with 12 ml of DMF containing 20% piperidine and further six times with 12 ml of DMF to remove the Fmoc group, then added with Fmoc-Phe-OH (0.387 g), PyBrop (0.466 g), NMP (12 ml) and DIPEA (0.523 ml), and shaken for 24 hours to form Fmoc-Phe-βEtAla-Alko resin. After filtration and washing with 12 ml of NMP, unreacted amino groups were capped by treatment with DMF (12 ml) containing 1-acetylimidazole (0.551 g) and DIPEA (0.174 ml) for 1 hour. Then, the resulting resin was washed again with 12 ml of NMP.

The Fmoc group was removed from the Fmoc-Phe-βEtAla-Alko resin in the same manner as described above. The resin was added with Fmoc-D-Arg(Pmc)-OH (0.707 g), HOBt (0.153 g), HBTU (0.379 g) and DIPEA (0.348 ml), and shaken for 1 hour to form Fmoc-D-Arg(Pmc)-Phe-βEtAla-Alko resin. After filtration and washing, unreacted amino groups were capped in the same manner as described above.

The Fmoc group was removed from the Fmoc-D-Arg(Pmc)-Phe-βEtAla-Alko resin in the same manner as described above, and the resultant was added with Fmoc-Tyr(t-Bu)-OH (0.460 g), HOBt (0.153 g), HBTU (0.399 g) and DIPEA (0.348 ml), and shaken for 1 hour to form Fmoc-Tyr(t-Bu)-D-Arg(Pmc)-Phe-βEtAla-Alko resin. Alter filtration and washing, unreacted amino groups were capped in the same manner as described above. The peptide was separated from the resin, and the protective group was simultaneously removed in the same manner as in the above (1).

(5) H-Tyr(Bzl)-D-Arg($Z_2$)-Phe-OTce

The starting material, Z-Phe-OTce (254 g), was treated with 25% hydrogen bromide-acetic acid (900 ml) to remove Z group and then dissolved in $CH_2Cl_2$ (1000 ml) on an ice bath. After this solution was added with Boc-D-Arg($Z_2$)-OH (288 g) and HOBt (85 g) and neutralized with TEA (77 ml), condensation reaction was carried out by using EDC • HCl (121 g) to form Boc-D-Arg($Z_2$)-Phe-OTce. Then, Boc-D-Arg($Z_2$)-Phe-OTce (241 g) was treated with 4N HCl/ethyl acetate (1,000 ml) to remove the Boc group, and dissolved in DMF (1,300 ml) on an ice bath. Alter the solution was added with Boc-Tyr(Bzl)-OH (108 g) and HOBt (46 g) and neutralized with TEA (42 ml), condensation reaction was carried out using EDC • HCl (65 g) to obtain a protected peptide represented by the following formula: Boc-Tyr(Bzl)-D-Arg($Z_2$)-Phe-OTce. The Boc-Tyr(Bzl)-D-Arg($Z_2$)-Phe-OTce (48 g) was treated with 4N HCl/ethyl acetate (250 ml) to remove Boc group.

(6) H-Tyr(Bzl)-D-Arg($Z_2$)-MePhe-MeβAla-OBzl

Using TosOH • MeβAla-OBzl as a starting material, the above peptide was prepared successively from the C-terminals by a liquid phase method. Boc-MePhe-OH and TosOH • MeβAla-OBzl were condensed by the EDC-HOBt method to afford Boc-MePhe-MeβAla-OBzl. Boc group was removed from the Boc-MePhe-MeβAla-OBzl using 4N HCl/ethyl acetate, and the resultant was condensed with Boc-D-Arg($Z_2$)-OH by the EDC-HOBt method to form Boc-D-Arg($Z_2$)-MePhe-MeβAla-OBzl. Subsequently, Boc group was removed from the Boc-D-Arg($Z_2$)-MePhe-MeβAla-OBzl using 4N HCl/ethyl acetate, and the resultant was condensed with Boc-Tyr(Bzl)-OH by the EDC-HOBt method to obtain the protected peptide Boc-Tyr(Bzl)-D-Arg($Z_2$)-MePhe-MeβAla-OBzl. Boc group was then removed by treating with 4N HCl/acetic acid (250 ml).

(B) Preparations of the compounds of the invention

Example 1: $H_2$NC(NH)-Phe-D-Arg-Phe-MeβAla-OH • diacetate

(1) BZA-Phe-D-Arg($Z_2$)-Phe-MeβAla-OBzl

Boc-Phe-D-Arg($Z_2$)-Phe-MeβAla-OBzl (0.51 g, 0.5 mmol) was dissolved in 4N HCl/ethyl acetate solution (20 ml) and stirred at room temperature for 1 hour. The reaction mixture was added with diethyl ether, and precipitated crystals were collected by filtration. These crystals were dissolved in dimethylformamide (20 ml), and further 1-(N,N'-bis(benzyloxycarbonyl)amidino)pyrazole (0.18 g, 0.48 mmol) and triethylamine (0.08 ml, 0.6 mmol) were dissolved in the solution. This solution was stirred at room temperature for 18 hours. The reaction mixture was added with ethyl acetate (50ml), and washed with 1N hydrochloric acid and then with saturated aqueous sodium hydrogencarbonate. The solvent was evaporated under reduced pressure. The resulting oily product was purified by silica gel column chromatography (eluted with benzene:ethyl acetate = 4:1) to obtain 0.36 g of a colorless oily product.

(2) $H_2NC(NH)$-Phe-D-Arg-Phe-MeβAla-OH • diacetate

The protected peptide obtained in the above (1) (0.36 g, 0.29 mmol) was dissolved in acetic acid (10 ml) and added with 0.2 g of 5% Pd-C (water content: 50%) as catalyst. Catalytic reduction was carried out for 4 hours to remove the protective group. The catalyst was removed by filtration, and the solvent was concentrated under reduced pressure. The resulting residue was dissolved in 0.1 N acetic acid and the solution was lyophilized to obtain 200 mg of the title compound as white powder. FAB MS m/z: 597 ($M+H^+$)

$[\alpha]_D^{23}$ +17.0° (c = 1.00, 1N acetic acid)
Rf; 0.61 (n-BuOH:AcOH:$H_2O$:pyridine = 15:3:10:12)

Example 2: $H_2NC(NH)$-DMT-D-Arg-Phe-MeβAla-OH • diacetate

(1) BZA-DMT-D-Arg($Z_2$)-Phe-MeβAla-OBzl

Boc-DMT-D-Arg($Z_2$)-Phe-MeβAla-OBzl (0.74 g, 0.7 mmol) was dissolved in 4N HCl/ethyl acetate solution (20 ml), and stirred at room temperature for 0.5 hours. The reaction mixture was added with diethyl ether, and the precipitated crystals were collected by filtration. These crystals were dissolved in dimethylformamide (20 ml), and 1-(N,N'-bis(benzyloxycarbonyl)amidino)pyrazole (0.25 g, 0.67 mmol) and triethylamine (0.12 ml, 0.84 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure. The resulting oily product was then purified by silica gel column chromatography (eluted with benzene:ethyl acetate = 3:1) to give 0.06 g of a colorless oily product.

(2) N-$H_2NC(NH)$-DMT-D-Arg-Phe-MeβAla-OH • diacetate

The protected peptide obtained in the above (1) (0.06 g, 0.05 mmol) was dissolved in acetic acid (10 ml) and added with 0.2 g of 5% Pd-C (water content: 50%) as catalyst. Then, catalytic reduction was performed for 4 hours to remove the protective group. After the catalyst was removed by filtration, the solvent was concentrated under reduced pressure, and the residue was dissolved in 0.1N acetic acid. The solution was lyophilized to obtain 30 mg of the title compound as white powder (yield: 83%).

FAB MS m/z: 641($M+H^+$)
$[\alpha]_D^{23}$ +31.7° (c = 1.0, 1N acetic acid)
Rf; 0.63 (n-BuOH:AcOH:$H_2O$:pyridine = 15:3:10:12)

Example 3: $H_2NC(NH)$-PMPA-D-Arg-Phe-MeβAla-OH • diacetate

(1) BZA-PMPA-D-Arg($Z_2$)-Phe-MeβAla-OBzl

Boc-PMPA-D-Arg($Z_2$)-Phe-MeβAla-OBzl (0.5 g, 0.48 mmol) was dissolved in 4N HCl/ethyl acetate solution (20 ml), and stirred at room temperature for 1 hour. The reaction mixture was added with diethyl ether, and the precipitated crystals were collected by filtration. These crystals were dissolved in dimethylformamide (20ml), and 1-(N,N'-bis(benzyloxycarbonyl)amidino)pyrazole (0.17 g, 0.46 mmol) and triethylamine (0.08 ml, 0.57 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was then purified by silica gel column chromatography (eluted with benzene:ethyl acetate = 4:1) to give 0.22 g of a colorless oily product.

(2) N-$H_2NC(NH)$-PMPA-D-Arg-Phe-MeβAla-OH • diacetate

The protected peptide obtained in the above (1) (0.20 g, 0.16 mmol) was dissolved in acetic acid (10 ml), and added with 0.20 g of 5% Pd-C (water content: 50%) as catalyst. Then, catalytic reduction was carried out for 4 hours to remove the protective group. After the catalyst was removed by filtration, the solvent was concentrated under reduced pressure, and the resulting residue was dissolved in 0.1N acetic acid. The solution was lyophilized to obtain 80 mg of the title compound as white powder.

FAB MS m/z : 627 (M+H$^+$)
$[\alpha]_D^{23}$ +11.9° (c = 1.0, 1N acetic acid)
Rf; 0.61 (n-BuOH:AcOH:$H_2O$:pyridine = 15:3:10:12)

Example 4: $H_2NC(NH)$-DOPA-D-Arg-Phe-MeβAla-OH • diacetate

(1) BZA-DOPA-D-Arg($Z_2$)-Phe-MeβAla-OBzl

Boc-DOPA-D-Arg($Z_2$)-Phe-MeβAla-OBzl (0.32 g, 0.3 mmol) was dissolved in 4N HCl/ethyl acetate solution (20 ml), and stirred at room temperature for 1 hour. The reaction mixture was added with diethyl ether, and the resulting precipitated crystals were collected by filtration. The crystals were dissolved in dimethylformamide (20 ml), and 1-(N,N'-bis(benzyloxycarbonyl)amidino)pyrazole (0.10 g, 0.26 mmol) and triethylamine (0.05 ml, 0.33 mmol) were dissolved in the solution. This solution was stirred at room temperature for 18 hours. The reaction mixture was concentrated under reduced pressure, and the residue was added with ethyl acetate (50ml) and washed with 1N hydrochloric acid and then with saturated aqueous sodium hydrogencarbonate. The solvent was concentrated under reduced pressure, and the resulting oily product was then purified by silica gel column chromatography (eluted with benzene:ethyl acetate = 2:1) to give 0.19 g of a colorless oily product.

(2) N-$H_2NC(NH)$-DOPA-D-Arg-Phe-MeβAla-OH • diacetate

The protected peptide obtained in the above (1) (0.19 g, 0.15 mmol) was dissolved in acetic acid (10 ml), and added with 0.20 g of 5% Pd-C (water content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to give 70 mg of the title compound as white powder.

FAB MS m/z : 629 (M+H$^+$)
$[\alpha]_D^{23}$ +7.66° (c = 1.0, 1N acetic acid)
Rf; 0.58 (n-BuOH:AcOH:$H_2O$:pyridine = 15:3:10:12)

Example 5: $H_2NC(NH)$-Tyr($SO_3H$)-D-Arg-Phe-βAla-OH

(1) N-$H_2NC(NH)$-Tyr-D-Arg-Phe-βAla-OH • diacetate

N-BZA-Tyr(Bzl)-D-Arg($Z_2$)-Phe-βAla-OBzl was prepared by a liquid phase method or a solid phase method ordinarily used for the peptide synthesis Then, the protected peptide was dissolved in acetic acid as in Example 4 (2), and added with 5% Pd-C (water content: 50%) as catalyst, and then catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to obtain the title compound as white powder.

(2) N-$H_2NC(NH)$-Tyr($SO_3H$)-D-Arg-Phe-βAla-OH

The protected peptide obtained in the above (1) (0.13 g, 2 mmol) was added to dimethylformamide (1.54 g, 100 mmol) at room temperature, and the resulting solution was stirred at room temperature for 4 hours. Then, the solution was added with isopropyl ether (100 ml) and stirred for a few minutes. After the solvent was removed by decantation, the solution was again washed by adding isopropyl ether (100 ml). The oily product obtained was added with ice cooled aqueous ammonia (29%, 40 ml) and stirred for 1 hour with ice cooling. The resulting crude product was dissolved in water, and purified by ODS column chromatography (eluted with water:methanol = 75:25). The desired fractions were collected and lyophilized to give 53 mg of the title compound as white powder.

FAB MS m/z : 678 (M+H$^+$)
Rf; 0.57 (n-BuOH:AcOH:$H_2O$:pyridine = 15:3:10:12)

Example 6: $H_2NC(NH)$-Hyp-D-Arg-Phe-MeβAla-OH • diacetate

(1) N-BZA-Hyp-D-Arg($Z_2$)-Phe-MeβAla-OBzl

Boc-Hyp-D-Arg($Z_2$)-Phe-MeβAla-OBzl (0.49 g, 0.5 mmol) was dissolved in 4N HCl/ethyl acetate solution (20 ml) and stirred at room temperature for 1 hour. The reaction mixture was added with diethyl ether, and the precipitated crystals were collected by filtration. The crystals were dissolved in dimethylformamide (20 ml), and 1-(N,N'-bis(benzyloxycarbonyl)amidino)pyrazole (0.23 g, 0.61 mmol) and triethylamine (0.07 ml, 0.50 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was then purified by silica gel column chromatography (eluted with chloroform:methanol = 100:1) to give 0.40 g of a colorless oily product.

(2) N-$H_2NC(NH)$-Hyp-D-Arg-Phe-MeβAla-OH • diacetate

The protected peptide obtained in the above (1) (0.2 g, 0.17 mmol) was dissolved in acetic acid (10 ml) and added with 0.20 g of 5% Pd-C (water content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to give 100 mg of the title compound as white powder.

FAB MS m/z : 563 ($M+H^+$)
$[\alpha]_D^{23}$= +14.8° (c= 1.13, 1N acetic acid)
Rf: 0.44 (n-BuOH:AcOH:$H_2O$:pyridine = 15:3:10:12)

Example 7: $H_2NC(NH)$-Tyr-D-Lys-Phe-MeβAla-OH • diacetate

(1) N-BZA-Tyr(Bzl)-D-Lys(Z)-Phe-MeβAla-OBzl

Boc-Tyr(Bzl)-D-Lys(Z)-Phe-MeβAla-OBzl (7.65 g, 8.00 mmol) was dissolved in 4N HCl/ethyl acetate solution (20 ml) and stirred at room temperature for 1 hour. The reaction mixture was added with diethyl ether, and the precipitated crystals were collected by filtration. The crystals were dissolved in dimethylformamide (20 ml), and 1-(N,N'-bis(benzyloxycarbonyl)amidino)pyrazole (3.33 g, 8.80 mmol) and triethylamine (1.12 ml, 8.00 mmol) were dissolved in the solution. This solution was stirred at room temperature for 50 hours. The reaction mixture was added with 10% aqueous solution of citric acid (100 ml), and the supernatant was removed by decantation. The deposited oily product was dissolved in chloroform (40 ml) and washed with saturated aqueous sodium hydrogencarbonate. The solvent was concentrated under reduced pressure, and the resulting oily product was crystallized from hexane to afford 6.81 g of white crystals. mp 136-138°C.

(2) N-$H_2NC(NH)$-Tyr-D-Lys-Phe-MeβAla-OH • diacetate

The protected peptide (1.50 g, 1.29 mmol) obtained in the above (1) was dissolved in acetic acid (10 ml) and added with 0.75 g of 5% Pd-C (water content: 50%) as catalyst. Then, catalytic reduction was carried out for 3.5 hours to remove the protective group. After the catalyst was removed by filtration, the solvent was concentrated under reduced pressure, and the resulting residue was charged on an ODS chromatography column (Fuji Silysia, DM 1020T, 50g) and eluted stepwise with a gradient of 1-10% acetonitrile/0.1N acetic acid solution. Fractions containing the desired compound was collected and lyophilized to obtain 520 mg of the title compound as white powder.

FAB MS m/z : 584 ($M+H^+$)
$[\alpha]_D^{23}$ +33.9° (c = 1.05, 1N acetic acid)
Rf; 0.54 (n-BuOH:AcOH:$H_2O$:pyridine = 15:3:10:12)

Example 8: $H_2NC(NH)$-Tyr-(R)-AGHX-Phe-MeβAla-OH • diacetate

$H_2NC(NH)$-Tyr-D-Lys-Phe-MeβAla-OH • diacetate obtained in Example 7 (2) (500 mg, 0.71 mmol) was dissolved in a mixture of dimethylformamide (4 ml) and water (3 ml), and then 1H-pyrazole-1-carboxyamidine hydrochloride (1.08 g, 0.74 mmol) and triethylamine (0.35 ml, 2.5 mmol) were dissolved in the solution. This solution was stirred at room temperature for 20 hours. The pH of the reaction mixture was adjusted to 4 by adding acetic acid, and the mixture was concentrated under reduced pressure. Acetic acid salt of the resulting crude product was charged on an ODS chromatography column (Fuji Silysia DM 1020T, 25 g) and eluted stepwise with a gradient of 1-10% acetonitrile/0.1N acetic acid solution. Fractions containing the desired product were collected and lyophilized to obtain 228 mg of the title compound as white powder.

FAB MS m/z : 626 ($M+H^+$)
$[\alpha]_D^{23}$ +27.5° (c = 1.05, 1N acetic acid)
Rf; 0.58 (n-BuOH:AcOH:$H_2O$:pyridine = 15:3:10:12)

Example 9: $H_2NC(NH)$-Tyr-(R)-DABA-Phe-MeβAla-OH • diacetate

Using Boc-Tyr(Bzl)-(R)-DABA(Z)-Phe-MeβAla • OBzl (7.07 g, 7.62 mmol), 1-(N,N'-bis(benzyloxycarbonyl)amidino)pyrazole (3.17 g, 8.38 mmol), and triethylamine (1.40 ml, 10.0 mmol), reactions were carried out as in Example 7. The resulting crude product was charged on an ODS chromatography column (Fuji Silysia DM 1020T, 100 g) and eluted stepwise with a gradient of 1-9% acetonitrile/0.1N acetic acid solution. Fractions containing the desired product were collected and lyophilized to obtain 1.06 g of the title compound as white powder.

FAB MS m/z : 557 ($M+H^+$)
$[\alpha]_D^{23}$ +34.0° (c = 1.05, 1N acetic acid)
Rf; 0.58 (n-BuOH:AcOH:$H_2O$:pyridine = 15:3:10:12)

Example 10: $H_2NC(NH)$-Tyr-(R)-AGBA-Phe-MeβAla-OH • diacetate

Using $H_2NC(NH)$-Tyr-(R)-DABA-Phe-MeβAla-OH • diacetate obtained in Example 9 (2.00 g, 2.96 mmol), 1H-pyrazole-1-carboxyamidine hydrochloride (1.39 g, 9.49 mmol), and triethylamine (1.82 ml, 13.0 mmol), reaction were carried out as in Example 8. The resulting crude product was charged on an ODS chromatography column (Fuji Silysia DM 1020T, 100 g) and eluted stepwise with a gradient of 1-9% acetonitrile/0.1N acetic acid solution. Fractions containing the desired product were collected and lyophilized to obtain 1.45 g of the title compound as white powder.

FAB MS m/z : 584 ($M+H^+$)
$[\alpha]_D^{23}$ +28.1° (c= 1.01, 1N acetic acid)
Rf; 0.60 (n-BuOH:AcOH:$H_2O$:pyridine = 15:3:10:12)

Example 11: $H_2NC(NH)$-Tyr-(R)-DAPR-Phe-MeβAla-OH • diacetate

Using Boc-Tyr(Bzl)-(R)-DAPR(Z)-Phe-MeβAla-OBzl (6.01 g, 6.58 mmol), 1-(N,N'-bis(benzyloxycarbonyl)amidino)pyrazole (2.74 g, 7.24 mmol), and triethylamine (1.40 ml, 10.0 mmol), reactions were carried out as in Example 7. The resulting crude product was charged on an ODS chromatography column (Fuji Silysia DM 1020T, 100 g) and eluted stepwise with a gradient of 1-8% acetonitrile/0.1N acetic acid solution. Fractions containing the desired product were collected and lyophilized to obtain 668 mg (50.0%) of the title compound as white powder.

FAB MS m/z : 542 ($M+H^+$)
$[\alpha]_D^{23}$ +24.6° (c = 1.04, 1N acetic acid)
Rf; 0.58 (n-BuOH:AcOH:$H_2O$:pyridine = 15:3:10:12)

Example 12: $H_2NC(NH)$-Tyr-(R)-AGPR-Phe-MeβAla-OH • diacetate

Using $H_2NC(NH)$-Tyr-(R)-DAPR-Phe-MeβAla-OH • diacetate obtained in Example 11 (1.80 g, 2.72 mmol), 1H-pyrazole-1-carboxyamidine hydrochloride (1.28 g, 8.72 mmol), and triethylamine (2.10 ml, 15.0 mmol), reactions were carried out as in Example 8. The resulting crude product was charged on an ODS chromatography column (Fuji Silysia DM 1020T, 100 g) and eluted stepwise with a gradient of 1-7% acetonitrile/0.1N acetic acid solution. Fractions containing the desired product were collected and lyophilized to obtain 1.01 g of the title compound as white powder.

FAB MS m/z : 585 ($M+H^+$)
$[\alpha]_D^{23}$ +20.3° (c = 1.01, 1N acetic acid)
Rf; 0.60 (n-BuOH:AcOH:$H_2O$:pyridine = 15:3:10:12)

Example 13: $H_2NC(NH)$-Tyr-(R)-PAPA-Phe-MeβAla-OH • diacetate

Using Boc-Tyr(Bzl)-(R)-PAPA(Z)-Phe-MeβAla-OBzl (3.30 g, 3.32 mmol), 1-(N,N'-bis(benzyloxycarbonyl)amidino)pyrazole (1.36 g, 3.60 mmol), and diisopropylethylamine (1.05 ml, 6.00 mmol), reactions were carried out as in Example 7. The resulting crude product was charged on an ODS chromatography column (Fuji Silysia DM 1020T, 190 g) and eluted stepwise with a gradient of 6-15% acetonitrile/0.1N acetic acid solution. Fractions containing the desired product were collected and lyophilized to obtain 1.36 g of the title compound as white powder.

FAB MS m/z : 619 ($M+H^+$)
$[\alpha]_D^{23}$ +5.47° (c = 0.99, 1N acetic acid)
Rf; 0.63 (n-BuOH:AcOH:$H_2O$:pyridine = 15:3:10:12)

Example 14: $H_2NC(NH)$-Tyr-(R)-PGPA-Phe-MeβAla-OH • diacetate

Using N-$H_2NC(NH)$-Tyr-(R)-PAPA-Phe-MeβAla-OH • diacetate obtained in Example 13 (0.60 g, 0.80 mmol), 1H-pyrazole-1-carboxyamidine hydrochloride (0.75 g, 3.98 mmol), and triethylamine (0.78 ml, 0.70 mmol), reactions were carried out as in Example 8. The resulting crude product was charged on an ODS chromatography column (Fuji Silysia DM 1020T, 75 g) and eluted stepwise with a gradient of 3-9% acetonitrile/0.1N acetic acid solution. Fractions containing the desired product were collected and lyophilized to obtain 208 mg (26.0%) of the title compound as white powder.

FAB MS m/z : 661 ($M+H^+$)
$[\alpha]_D^{23}$ -2.05° (c = 1.09, 1N acetic acid)
Rf; 0.63 (n-BuOH:AcOH:$H_2O$:pyridine = 15:3:10:12)

Example 15: N-$H_2NC(NH)$-Tyr-D-Orn-Phe-MeβAla-OH • diacetate

(1) BZA-Tyr(Bzl)-D-Orn(Troc)-Phe-MeβAla-OBzl

Boc-Tyr(Bzl)-D-Orn(Troc)-Phe-MeβAla-OBzl (5.20 g, 5.29 mmol) was dissolved in 4N HCl/ethyl acetate solution (30 ml) and stirred at room temperature for 1.5 hours. The reaction mixture was added with diethyl ether, and the precipitated crystals were collected by filtration. The crystals were dissolved in dimethylformamide (25 ml), and then 1-(N,N'-bis(benzyloxycarbonyl)amidino)pyrazole (2.40 g, 6.35 mmol) and triethylamine (0.73 ml, 5.3 mmol) were dissolved in the solution. This solution was stirred at room temperature for 18 hours. The reaction mixture was added with 10% aqueous solution of citric acid (100 ml) and the supernatant was removed by decantation. The deposited oily product was dissolved in chloroform (40 ml) and washed with saturated aqueous sodium hydrogencarbonate. The solvent was concentrated under reduced pressure, and the resulting oily product was purified by silica gel column chromatography (eluted with benzene:ethyl acetate = 3:1) to give 6.3 g of a colorless oily product.

(2) $H_2NC(NH)$-Tyr-D-Orn-Phe-MeβAla-OH

BZA-Tyr(Bzl)-D-Orn(Troc)-Phe-MeβAla-OBzl obtained in the above (1) was dissolved in 90% acetic acid (100 ml), added with zinc powder (6.70 g, 103 mmol) and stirred at room temperature for 2 hours. After the zinc powder was removed by filtration, 6.15 g of 5% Pd-C (water content: 50%) was added as catalyst and catalytic reduction was carried out for 5 hours. The catalyst was removed by filtration, and the solvent was concentrated under reduced pressure. The residue was charged on an ODS chromatography column (Fuji Silysia DM 1020T, 160 g) and eluted stepwise with a gradient of 1-9% acetonitrile/0.1N acetic acid solution. Fractions containing the desired product were collected and lyophilized to obtain 700 mg of white powder.

FAB MS m/z : 571 ($M+H^+$)
$[\alpha]_D^{23}$ +35.1° (c = 0.96, 1N acetic acid)
Rf; 0.58 (n-BuOH:AcOH:$H_2O$:pyridine = 15:3:10:12)

Example 16: $H_2NC(NH)$-Tyr-(R)-MGPE-Phe-MeβAla-OH • diacetate

(1) BZA-Tyr(Bzl)-(R)-MGPE(Z)-Phe-MeβAla-OBzl

Using N-BZA-Tyr(Bzl)-D-Orn(Troc)-Phe-MeβAla-OBzl (3.40 g, 2.85 mmol) obtained in Example 15 (1) was dissolved in 90% acetic acid (50 ml), added with zinc powder (3.75 g, 57.3 mmol), and stirred at room temperature for 2

hours. After the zinc powder was removed by filtration, the filtrate was concentrated under reduced pressure. The residue was added with chloroform (50 ml) and washed with saturated aqueous sodium hydrogencarbonate. Alter evaporating the solvent under reduced pressure, the residue was dissolved in dimethylformamide (15 ml), and then N-benzyloxycarbonyl-N'-methylthiourea (0.87 g, 3.89 mmol), 1-ethyl-3-(3-dimethylaminopropyl)carbodiimide hydrochloride (0.75 g, 3.89 mmol), and triethylamine (0.54 ml, 3.89 mmol) were dissolved with stirring under ice cooling. This solution was stirred at room temperature for 16 hours. The reaction mixture was added with ethyl acetate (100 ml), and washed with 10% aqueous solution of citric acid and saturated aqueous sodium hydrogencarbonate. The solvent was concentrated under reduced pressure, and the resulting oily product was then purified by silica gel column chromatography (eluted with chloroform:methanol = 70:1) to give 1.09 g of a colorless oily product.

(2) N-$H_2$NC(NH)-Tyr-(R)-MGPE-Phe-MeβAla-OH • diacetate

The protected peptide obtained in the above (1) (0.99 g, 0.82 mmol) was dissolved in acetic acid (10 ml) and added with 1.0 g of 5% Pd-C (water content: 50%) as catalyst. Catalytic reduction was carried out for 12.5 hours to remove the protective group. After the catalyst was removed by filtration, the filtrate was lyophilized to obtain 478 mg of the title compound as white powder.

FAB MS m/z : 626 (M+$H^+$)
$[\alpha]_D^{23}$ +23.5° (c= 1.04, 1N acetic acid)
Rf; 0.63 (n-BuOH:AcOH:$H_2$O:pyridine = 15:3:10:12)

Example 17: $H_2$NC(NH)-Tyr-(R)-IEPE-Phe-MeβAla-OH • diacetate

$H_2$NC(NH)-Tyr-D-Orn-Phe-MeβAla-OH obtained in Example 15 (1.00 g, 1.45 mmol) and ethyl acetimidate hydrochloride (358 mg, 2.90 mmol) were dissolved in dimethylformamide (10 ml), added with triethylamine (0.80 ml, 5.8 mmol), and then stirred at room temperature for 7 hours. The reaction mixture was added with diethyl ether (100 ml), and the solvent was removed by decantation. The resulting crude product was charged on an ODS chromatography column (Fuji Silysia DM 1020T, 100 g), and eluted stepwise with a gradient of 2-10% acetonitrile/0.1N acetic acid solution. Fractions containing the desired product were collected and lyophilized to obtain 378 mg of the title compound as white powder.

FAB MS m/z : 612 (M+$H^+$)
$[\alpha]_D^{23}$ +29.3° (c = 1.09, 1N acetic acid)
Rf; 0.58 (n-BuOH:AcOH:$H_2$O:pyridine = 15:3:10:12)

Example 18: $H_2$NC(NH)-Tyr-(R)-IPPE-Phe-MeβAla-OH • monoacetate

BZA-Tyr(Bzl)-D-Orn(Troc)-Phe-MeβAla-OBzl obtained in Example 15(1) (3.20 g, 2.68 mmol) was dissolved in acetic acid (50 ml), added with zinc powder (3.53 g, 54.0 mmol), and stirred at room temperature for 2 hours. After the zinc powder was removed by filtration, the filtrate was concentrated under reduced pressure. The residue was added with chloroform (50 ml) and washed with saturated aqueous sodium hydrogencarbonate. The solvent was evaporated under reduced pressure, and the residue was dissolved in methanol (10 ml), and then acetone (181 μl, 2.46 mmol) and sodium cyanoborohydride (179 mg, 2.70 mmol) were further dissolved in the solution with stirring under ice cooling. This solution was stirred at room temperature for 16 hours. The reaction mixture was concentrated under reduced pressure and the residue was added with chloroform (30 ml) and washed with 10% aqueous solution of citric acid and saturated aqueous sodium hydrogencarbonate. The solvent was concentrated under reduced pressure to obtain 2.5 g of a colorless oily product.

This protected peptide was dissolved in acetic acid (30 ml) and added with 2.5 g of 5% Pd-C (water content: 50%) as catalyst. Catalytic reduction was carried out for 6 hours to remove the protective group. After the catalyst was removed by filtration, the solvent was concentrated under reduced pressure, and then the residue was charged on an ODS chromatography column (Fuji Silysia DM 1020T, 100 g) and eluted stepwise with a gradient of 3-10% acetonitrile/0.1N acetic acid solution. Fractions containing the desired product were collected and lyophilized to give 1.2 g of the title compound as white powder.

FAB MS m/z : 612 (M+$H^+$)
$[\alpha]_D^{23}$ +27.9° (c = 1.09, 1N acetic acid)
Rf; 0.63 (n-BuOH:AcOH:$H_2$O:pyridine = 15:3:10:12)

Example 19: $H_3C$-C(NH)-Tyr-D-Lys-Phe-MeβAla-OH • diacetate

(1) $H_3C$-C(NH)-Tyr(Bzl)-D-Lys(Z)-Phe-MeβAla-OBzl

Boc-Tyr(Bzl)-D-Lys(Z)-Phe-MeβAla-OBzl (1.00 g, 1.05 mmol) was dissolved in 4N HCl/ethyl acetate solution (3 ml) and stirred at room temperature for 30 minutes. The reaction mixture was concentrated under reduced pressure, and the residue was dissolved in dimethylformamide (4 ml), and then ethyl acetimidate hydrochloride (0.26 g, 2.09 mmol) and triethylamine (0.28 ml, 2.00 mmol) were dissolved in the solution. This solution was stirred at room temperature for 20 hours. The reaction mixture was dissolved in ethyl acetate (50 ml) and washed with 10% aqueous solution of citric acid and saturated aqueous sodium hydrogencarbonate. The solvent was concentrated under reduced pressure, and the resulting oily product was then purified by silica gel column chromatography (eluted with chloroform:methanol = 20:1) to give 330 mg of a colorless oily product.

(2) $H_3C$-C(NH)-Tyr-D-Lys-Phe-MeβAla-OH • diacetate

The protected peptide obtained in the above (1) (248 mg, 0.76 mmol) was dissolved in acetic acid (4 ml) and added with 0.13 g of 5% Pd-C (water content: 50%) as catalyst. Catalytic reduction was carried out for 4.5 hours to remove the protective group. After the catalyst was removed by filtration, the solvent was concentrated under reduced pressure, and the resulting residue was charged on an ODS chromatography column (Fuji Silysia DM 1020T, 20 g) and eluted stepwise with a gradient of 1-7% acetonitrile/0.1N acetic acid solution. Fractions containing the desired product were collected and lyophilized to obtain 95 mg of the title compound as white powder.

FAB MS m/z : 584 ($M+H^+$)
$[\alpha]_D^{23}$ +26.4° (c= 1.05, 1N acetic acid)
Rf; 0.58 (n-BuOH:AcOH:$H_2O$:pyridine = 15:3:10:12)

Example 20: $H_2NC$(NH)-Tyr-D-Ala-Phe-MeβAla-OH • monoacetate

(1) N-BZA-Tyr(Bzl)-D-Ala-Phe-MeβAla-OBzl

Boc-Tyr(Bzl)-D-Ala-Phe-MeβAla-OBzl (0.39 g, 0.5 mmol) was dissolved in 4N HCl/ethyl acetate solution (30 ml), and stirred at room temperature for 30 minutes. The reaction mixture was added with diethyl ether, and the precipitated crystals were collected by filtration. These crystals were dissolved in dimethylformamide (25 ml), and then 1-(N,N'-bis(benzyloxycarbonyl)amidino)pyrazole (0.18 g, 0.48 mmol) and triethylamine (0.09 ml, 0.65 mmol) were dissolved in the solution. This solution was stirred at room temperature for 18 hours and concentrated under reduced pressure. Then, the residue was added with ethyl acetate (50 ml) and washed with 1N hydrochloric acid and then with saturated aqueous sodium hydrogencarbonate. The solvent was concentrated under reduced pressure, and the resulting oily product was purified by silica gel column chromatography (eluted with benzene:ethyl acetate = 5:2) to give 0.4 g of a colorless oily product.

(2) N-$H_2NC$(NH)-Tyr-D-Ala-Phe-MeβAla-OH • monoacetate

The protected peptide obtained in the above (1) (0.4 g, 0.41 mmol) was dissolved in acetic acid (10 ml) and added with 0.4 g of 5% Pd-C (water content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to afford 210 mg of the title compound as white powder.

FAB MS m/z : 528 ($M+H^+$)
$[\alpha]_D^{23}$ +38.2° (c = 1.0, 1N acetic acid)
Rf; 0.68 (n-BuOH:AcOH:$H_2O$:pyridine = 15:3:10:12)

Example 21: $H_2NC$(NH)-Tyr-D-Glu-Phe-MeβAla-OH • monoacetate

(1) BZA-Tyr(Bzl)-D-Glu(Bzl)-Phe-MeβAla-OBzl

Boc-Tyr(Bzl)-D-Glu(Bzl)-Phe-MeβAla-OBzl (0.46 g, 0.50 mmol) was dissolved in 4N HCl/ethyl acetate solution (30 ml), and stirred at room temperature for 1 hour. The reaction mixture was added with diethyl ether, and the precipitated

crystals were collected by filtration. These crystals were dissolved in dimethylformamide (5 ml), and then 1-(N,N'-bis(benzyloxycarbonyl)amidino)pyrazole (0.23 g, 0.6 mmol) and triethylamine (0.07 ml, 0.5 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was purified by silica gel column chromatography (eluted with chloroform) to give 0.39 g of a colorless oily product.

(2) $H_2NC(NH)$-Tyr-D-Glu-Phe-MeβAla-OH • monoacetate

The protected peptide obtained in the above (1) (0.2 g, 0.18 mmol) was dissolved in acetic acid (30 ml), and added with 0.2 g of 5% Pd-C (water content: 50%) as catalyst. Then, catalytic reduction was carried out for 3 hours to remove the protective group. After the catalyst was removed by filtration, the solvent was concentrated under reduced pressure. The resulting residue was dissolved in 0.1N acetic acid, and the solution was lyophilized to afford 102 mg of the title compound as white powder.

FAB MS m/z : 585 $(M+H^+)$
$[\alpha]_D^{23}$ +28.9° (c = 1.01, 1N acetic acid)
Rf; 0.61 (n-BuOH:AcOH:$H_2O$:pyridine = 15:3:10:12)

Example 22: $H_2NC(NH)$-Tyr-D-Nva-Phe-MeβAla-OH • monoacetate

(1) BZA-Tyr(Bzl)-D-Nva-Phe-MeβAla-OBzl

Boc-Tyr(Bzl)-D-Nva-Phe-MeβAla-OBzl (0.56 g, 0.7 mmol) was dissolved in 4N HCl/ethyl acetate solution (30 ml), and stirred at room temperature for 1.5 hours. The reaction mixture was added with diethyl ether, and the precipitated crystals were collected by filtration. These crystals were dissolved in dimethylformamide (25 ml), and then 1-(N,N'-bis(benzyloxycarbonyl)amidino)pyrazole (0.23 g, 0.6 mmol) and triethylamine (0.12 ml, 0.84 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was then purified by silica gel column chromatography (eluted with chloroform:methanol = 100:1) to give 0.42 g of a colorless oily product.

(2) $H_2NC(NH)$-Tyr-D-Nva-Phe-MeβAla-OH • monoacetate

The protected peptide (0.42 g, 0.42 mmol) obtained in the above (1) was dissolved in acetic acid (30 ml), and added with 0.2 g of 5% Pd-C (water content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to obtain 220 mg of the title compound as white powder.

FAB MS m/z : 556 $(M+H^+)$
$[\alpha]_D^{23}$ +33.7° (c = 1.01, 1N acetic acid)
Rf; 0.69 (n-BuOH:AcOH:$H_2O$:pyridine = 15:3:10:12)

Example 23: $H_2NC(NH)$-Tyr-D-Pro-Phe-MeβAla-OH • monoacetate

(1) BZA-Tyr(Bzl)-D-Pro-Phe-MeβAla-OBzl

Boc-Tyr(Bzl)-D-Pro-Phe-MeβAla-OBzl (0.4 g, 0.5 mmol) was dissolved in 4N HCl/ethyl acetate solution (20 ml), and stirred at room temperature for 1.5 hours. The reaction mixture was washed with hexane to give an oily product. The oily product was dissolved in dimethylformamide (5 ml), and then 1-(N,N'-bis(benzyloxy-carbonyl)amidino)pyrazole (0.23 g, 0.6 mmol) and triethylamine (0.07 ml, 0.23 mmol) were dissolved. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was purified by silica gel column chromatography (eluted with chloroform) to obtain 0.41 g of colorless oily product.

(2) $H_2NC(NH)$-Tyr-D-Pro-Phe-MeβAla-OH • monoacetate

The protected peptide (0.2 g, 0.2 mmol) obtained in the above (1) was dissolved in acetic acid (50 ml), and added with 0.2 g of 5% Pd-C (water content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to obtain 103 mg of the title compound as white powder.

FAB MS m/z : 554 $(M+H^+)$
$[\alpha]_D^{23}$ +56.1° (c = 1.06, 1N acetic acid)
Rf; 0.65 (n-BuOH:AcOH:$H_2O$:pyridine = 15:3:10:12)

Example 24: $H_2NC(NH)$-Tyr-D-(N-Me)Arg-Phe-MeβAla-OH • diacetate

(1) BZA-Tyr(Bzl)-D-(N-Me)Arg(Tos)-Phe-MeβAla-OBzl

Boc-Tyr(Bzl)-D-(N-Me)Arg(Tos)-Phe-MeβAla-OBzl (1.09 g, 1.1 mmol) was dissolved in 4N HCl/ethyl acetate solution (20 ml), and stirred at room temperature for 1 hour. The reaction mixture was added with diethyl ether, and the precipitated crystals were collected by filtration. These crystals were dissolved in dimethylformamide (25 ml), and then 1-(N,N'-bis(benzyloxycarbonyl)amidino)pyrazole (0.22 g 0.59 mmol) and triethylamine (0.07 ml, 0.50 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was purified by silica gel column chromatography (eluted with benzene:ethyl acetate = 1:2) to give 0.4 g of a colorless oily product.

(2) $H_2NC(NH)$-Tyr-D-(N-Me)Arg-Phe-MeβAla-OH • diacetate

The protected peptide obtained in the above (1) (0.3 g, 0.24 mmol) was dissolved in acetic acid (30 ml), and added with 0.3 g of 5% Pd-C (water content: 50%) as catalyst. Then, catalytic reduction was carried out for 3 hours to remove the protective group. After the catalyst was removed by filtration, the solvent was concentrated under reduced pressure. The residue was dissolved in 0.1N acetic acid, and the solution was lyophilized to give 170 mg of the title compound as white powder (yield: 83%). This powder was added with anisole (2 ml) and allowed to react with hydrogen fluoride for 30 minutes. After hydrogen fluoride was removed under reduced pressure, the residue was washed with diethyl ether several times to give powdery residue. The residue was dissolved in 0.1N acetic acid, and the solution was charged on a column filled with anion exchange resin PA 308, and eluted with water. Fractions containing the desired product were collected and lyophilized to obtain 109 mg of white powder.

FAB MS m/z : 627 $(M+H^+)$
$[\alpha]_D^{23}$ +37.9° (c = 1.03, 1N acetic acid)
Rf ; 0.59 (n-BuOH:AcOH:$H_2O$:pyridine = 15:3:10:12)

Example 25: $H_2NC(NH)$-Tyr-D-Arg-Tyr-MeβAla-OH • diacetate

(1) BZA-Tyr(Bzl)-D-Arg($Z_2$)-Tyr(Bzl)-MeβAla-OBzl

Boc-Tyr(Bzl)-D-Arg($Z_2$)-Tyr(Bzl)-MeβAla-OBzl (0.61 g, 0.50 mmol) was dissolved in 4N HCl/ethyl acetate solution (20 ml), and stirred at room temperature for 1 hour. The reaction mixture was added with diethyl ether, and the precipitated crystals were collected by filtration. These crystals were dissolved in dimethylformamide (10 ml), and then 1-(N,N'-bis(benzyloxycarbonyl)amidino)pyrazole (0.23 g, 0.6 mmol) and triethylamine (0.07 ml, 0.5 mmol) were dissolved in the solution. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was then solidified from ethyl acetate/hexane to give 0.5 g of colorless semi-oily product.

(2) $H_2NC(NH)$-Tyr-D-Arg-Tyr-MeβAla-OH • diacetate

The protected peptide obtained in the above (1) (0.2 g, 0.14 mmol) was dissolved in acetic acid (20 ml), and added

with 0.2 g of 5% Pd-C (water content: 50%) as catalyst. Then, catalytic reduction was carried out for 3 hours to remove the protective group. After the catalyst was removed by filtration, the solvent was concentrated under reduced pressure. The residue was dissolved in 0.1N acetic acid, and the solution was lyophilized to obtain 98 mg of the title compound as white powder.

FAB MS m/z : 629 ($M+H^+$)
$[\alpha]_D^{23}$ +30.9° (c = 0.97, 1N acetic acid)
Rf; 0.53 (n-BuOH:AcOH:$H_2O$:pyridine = 15:3:10:12)

Example 26: N-$H_2$NC(NH)-Tyr-D-Arg-APBA-MeβAla-OH • diacetate

(1) N-BZA-Tyr(Bzl)-D-Arg($Z_2$)-APBA-MeβAla-OBzl

Boc-Tyr(Bzl)-D-Arg($Z_2$)-APBA-MeβAla-OBzl (0.34 g, 0.3 mmol) was dissolved in 4N HCl/ethyl acetate solution (20 ml), and stirred at room temperature for 0.5 hour. The reaction mixture was added with diethyl ether to give a washed oily product. This oily product was dissolved in dimethylformamide (20 ml), and then 1-(N,N' bis(benzyloxycarbonyl)amidino)pyrazole (0.10 g, 0.27 mmol) and triethylamine (0.05 ml, 0.36 mmol) were dissolved in the solution. The solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was purified by silica gel column chromatography (eluted with benzene:ethyl acetate = 3:1) to obtain 0.36 g of a colorless oily product.

(2) $H_2$NC(NH)-Tyr-D-Arg-APBA-MeβAla-OH • diacetate

The protected peptide (0.3 g, 0.22 mmol) obtained in the above (1) was dissolved in acetic acid (20 ml), and added with 0.2 g of 5% Pd-C (aqueous content: 50%) as catalyst. Then, catalytic reduction was carried out for 4 hours to remove the protective group. After the catalyst was removed by filtration, the solvent was concentrated under reduced pressure. The resulting residue was dissolved in 0.1N acetic acid, and the solution was lyophilized to obtain 130 mg of the title compound as white powder.

FAB MS m/z : 627 ($M+H^+$)
$[\alpha]_D^{23}$ +13.6° (c = 1.00, 1N acetic acid)
Rf; 0.63 (n-BuOH:AcOH:$H_2O$:pyridine = 15:3:10:12)

Example 27: $H_2$NC(NH)-Tyr-D-Arg-Cha-MeβAla-OH • diacetate

(1) BZA-Tyr(Bzl)-D-Arg($Z_2$)-Cha-MeβAla-OBzl

Boc-Tyr(Bzl)-D-Arg($Z_2$)-Cha-MeβAla-OBzl (0.56 g, 0.5 mmol) was dissolved in 4N HCl/ethyl acetate solution (20 ml), and stirred at room temperature for 1 hour. The reaction mixture was added with diethyl ether, and the precipitated crystals were collected by filtration. These crystals were dissolved in dimethylformamide (5 ml), and then 1-(N,N'-bis(benzyloxycarbonyl)amidino)pyrazole (0.2 g, 0.53 mmol) and triethylamine (0.06 ml, 0.2 mmol) were dissolved. This solution was stirred at room temperature for 18 hours. The reaction mixture was concentrated under reduced pressure, and the residue was added with ethyl acetate (50 ml) and washed with 1N hydrochloric acid and then with saturated aqueous sodium hydrogencarbonate. The solvent was concentrated under reduced pressure, and the resulting oily product was solidified from hexane to give 0.4 g of a colorless semi-oily product.

(2) $H_2$NC(NH)-Tyr-D-Arg-Cha-MeβAla-OH • diacetate

The protected peptide obtained in the above (1) (0.2 g, 0.15 mmol) was dissolved in acetic acid (20 ml), and added with 0.2 g of 5% Pd-C (water content: 50%) as catalyst. Then catalytic reduction was carried out for 3 hours to remove the protective group. After the catalyst was removed by filtration, the solvent was concentrated under reduced pressure, and the resulting residue was dissolved in 0.1N acetic acid. The solution was lyophilized to obtain 96 mg of the title compound as white powder.

FAB MS m/z : 619 ($M+H^+$)
$[\alpha]_D^{23}$ +17.9° (c = 0.98, 1N acetic acid)

Rf; 0.61 (n-BuOH:AcOH:$H_2O$:pyridine = 15:3:10:12)

Example 28: $H_2NC(NH)$-Tyr-D-AMSB-Phe-MeβAla-OH • monoacetate

(1) Boc-Tyr-D-AMSB-Phe-MeβAla-OMe

Boc-D-AMSB-Phe-MeβAla-OMe (0.86 g, 1.68 mmol) was dissolved in 4N HCl/ethyl acetate solution (30 ml), and stirred at room temperature for 30 minutes. The reaction mixture was concentrated under reduced pressure, and the residue was washed with diethyl ether. The solvent was concentrated under reduced pressure, and the resulting oily product was dissolved in methylene chloride (25 ml). Then, 1-hydroxybenzotriazole (0.27 g, 2.02 mmol) and triethylamine (0.28 ml, 2.02 mmol) were dissolved in the solution. This solution was stirred at room temperature for 18 hours. The reaction mixture was concentrated under reduced pressure, and the residue was added with ethyl acetate (50 ml) and washed with 1N HCl and then with 10% aqueous solution of sodium carbonate. The solvent was concentrated under reduced pressure, and the resulting residue was purified by silica gel column chromatography (eluted with chloroform:methanol = 50:1) to afford 0.17 g of colorless powder.

(2) Boc-Tyr-D-AMSB-Phe-MeβAla-OH

The protected peptide obtained in the above (1) (0.17 g, 0.25 mmol) was dissolved in methanol (20 ml), added with 1N sodium hydroxide solution (0.28 ml, 0.28 mmol), and stirred at room temperature for 6 hours. The methanol was concentrated under reduced pressure, the residue was dissolved in diethyl ether and water, and then the solution was separated. The aqueous layer was acidified with 0.5N HCl (pH 2-3), added with ethyl acetate (80 ml), and washed with water. The solvent was concentrated under reduced pressure, and the residue was solidified by adding hexane and collected by filtration to obtain 0.11 g of white powder.

(3) $H_2NC(NH)$-Tyr-D-AMSB-Phe-MeβAla-OH • monoacetate

The protected peptide (0.11 g, 0.17 mmol) obtained in the above (2) was dissolved in 4N HCl/ethyl acetate (30 ml), and stirred at room temperature for 30 minutes. The reaction mixture was added with diethyl ether, and the precipitated crystals were collected by filtration. These crystals were dissolved in dimethylformamide (25 ml), and then an amidination regent (1H-pyrazole-1-carboxamidine • hydrochloride, 0.03 g, 0.19 mmol) and triethylamine (0.10 ml, 0.72 mmol) were dissolved in the solution. This solution was stirred at room temperature for 18 hours. The reaction mixture was again added with amidination regent (1H-pyrazole-1-carboxamidine • hydrochloride, 0.015 g, 0.09 mmol) and triethylamine (0.05 ml, 0.36 mmol), and this solution was stirred at room temperature for 18 hours. The reaction mixture was washed twice by the addition of diethyl ether and successive decantation to give an oily product. The oily product was dissolved in water, adjusted to pH 4 with acetic acid, and purified by ODS column chromatography (eluted with acetonitrile/0.1N acetic acid) to give a colorless oily product. The oily product was dissolved in 0.1N acetic acid, and the solution was lyophilized to obtain 30 mg of the title compound as white powder.

FAB MS m/z: 603 $(M+H^+)$
Rf; 0.61 (n-BuOH:AcOH:$H_2O$:pyridine = 15:3:10:12)

Example 29: $H_2NC(NH)$-Tyr-D-Arg-Phe(p-Cl)-MeβAla-OH • diacetate

(1) BZA-Tyr(Bzl)-D-Arg($Z_2$)-Phe(p-Cl)-MeβAla-OBzl

Boc-Tyr(Bzl)-D-Arg($Z_2$)-Phe(p-Cl)-MeβAla-OBzl (0.58 g, 0.50 mmol) was dissolved in 4N HCl/ethyl acetate solution (20 ml), and stirred at room temperature for 0.5 hour. The reaction mixture was added with diethyl ether, and the precipitated crystals were collected by filtration. These crystals were dissolved in dimethylformamide (10 ml), and then 1-(N,N'-bis(benzyloxycarbonyl)amidino)pyrazole (0.23 g, 0.6 mmol) and triethylamine (0.07 ml, 0.5 mmol) were dissolved in the solution. This solution was stirred at room temperature for 18 hours. The reaction mixture was concentrated under reduced pressure, and the residue was added with ethyl acetate (50 ml) and washed with 1N HCl and then with 10% aqueous solution of sodium carbonate. The solvent was concentrated under reduced pressure, and the resulting oily product was purified by silica gel column chromatography (eluted with benzene:ethyl acetate = 4:1) to give 0.48 g of light yellow powder.

(2) $H_2NC(NH)$-Tyr-D-Arg-Phe(p-Cl)-MeβAla-OBzl • diacetate

The protected peptide obtained in the above (1) (0.2 g, 0.14 mmol) was added with anisole (0.6 ml), and the mixture was then added with hydrogen fluoride (5 ml) and stirred at 0 °C for 30 minutes. The reaction mixture was concentrated under reduced pressure, and the residue was washed with diethyl ether. The resulting powder was dissolved in 0.1N acetic acid, and the solution was treated with an ion exchange resin (PA-308, eluted with water) to afford acetate salt, which was then lyophilized to obtain 83 mg of the title compound as white powder.

FAB MS m/z : 647 ($M+H^+$)
$[\alpha]_D^{23}$ +25.3° (c=1.00, 1N acetic acid)
Rf; 0.55 (n-BuOH:AcOH:$H_2O$:pyridine = 15:3:10:12)

Example 30: $H_2NC(NH)$-Tyr-D-Arg-Phe(p-F)-MeβAla-OH • diacetate

(1) BZA-Tyr(Bzl)-D-Arg($Z_2$)-Phe(p-F)-MeβAla-OBzl

Boc-Tyr(Bzl)-D-Arg($Z_2$)-Phe(p-F)-MeβAla-OBzl (0.57 g, 0.50 mmol) was dissolved in 4N HCl/ethyl acetate solution (20 ml), and stirred at room temperature for 0.5 hour. The reaction mixture was added with diethyl ether, and the precipitated crystals were collected by filtration. These crystals were dissolved in dimethylformamide (10 ml), and then 1-(N,N'-bis(benzyloxycarbonyl)amidino)pyrazole (0.23 g, 0.6 mmol) and triethylamine (0.07 ml, 0.5 mmol) were dissolved in the solution. The solution was stirred at room temperature for 18 hours. The reaction mixture was concentrated under reduced pressure, and the residue was added with ethyl acetate (50 ml) and washed with 1N HCl and then with 10% aqueous sodium carbonate. The solvent was concentrated under reduced pressure, and the resulting oily product was purified by silica gel column chromatography (eluted with benzene:ethyl acetate = 4:1) to afford 0.44 g of light yellow powder.

(2) $H_2NC(NH)$-Tyr-D-Arg-Phe(p-F)-MeβAla-OBzl • diacetate

The protected peptide obtained in the above (1) (0.2 g, 0.15 mmol) was dissolved in acetic acid (20 ml), and added with 0.2 g of 5% Pd-C (aqueous content: 50%) as catalyst, and then catalytic reduction was carried out for 3 hours to remove the protective group. After the catalyst was removed by filtration, the solvent was concentrated under reduced pressure. The resulting residue was dissolved in 0.1N acetic acid, and the solution was lyophilized to obtain 90 mg of the title compound as white powder.

FAB MS m/z : 631 ($M+H^+$)
$[\alpha]_D^{23}$ +21.2° (c=1.00, 1N acetic acid)
Rf; 0.51 (n-BuOH:AcOH:$H_2O$:pyridine = 15:3:10:12)

Example 31: $H_3C$-C(NH)-Tyr-D-AMSB-Phe-MeβAla-OH • acetate

(1) Boc-Tyr-D-AMSB-Phe-MeβAla-OH

Boc-Tyr-D-Met-Phe-MeβAla-OMe (9.45 g, 14.3 mmol) was suspended in 67% methanol (110 ml), added with 1N NaOH (28.6 ml), and stirred at room temperature for 2 hours. The reaction solution was added with water (50 ml), and the methanol was concentrated under reduced pressure. The residue was washed with ethyl acetate. The pH of the aqueous layer was adjusted to 3 using 6N HCl under ice cooling. The deposited oily product was extracted with ethyl acetate and washed with saturated brine. The extract was dried over magnesium sulfate and concentrated under reduced pressure. The resulting oily product was dissolved in acetic acid (200 ml), added with 30% aqueous hydrogen peroxide (1.53 ml), and stirred at room temperature for 2 hours. After the solvent was removed by evaporation under reduced pressure, the residue was crystallized from diethyl ether to obtain 9.39 g of the desired compound as crystals (99.5%).

(2) $H_3C$-C(NH)-Tyr-D-AMSB-Phe-MeβAla-OH • acetate

Boc-Tyr-D-AMSB-Phe-MeβAla-OH obtained in the above (1) (9.37 g, 14.2 mmol) was dissolved in 4N HCl/dioxane (100 ml), and stirred at room temperature for 30 minutes. The reaction mixture was added with diethyl ether, and the precipitated crystals were collected by filtration. The crystals were dissolved in dimethylformamide (70 ml), and then ethyl acetimidate hydrochloride (3.51 g, 28.4 mmol) and triethylamine (8.4 ml, 60 mmol) were dissolved in the solution.

This solution was stirred at room temperature for 3 hours. The reaction mixture was added with diethyl ether, and the supernatant was removed by decantation. The resulting oily product was charged on an ODS chromatography column (Fuji Silysia DM 1020T, 200g) and eluted stepwise with a gradient of 2-11% acetonitrile/0.1N acetic acid solution. Fractions containing the desired product were collected and lyophilized to obtain 2.57 g of the title compound as white powder.

FAB MS m/z : 602 ($M+H^+$)
$[\alpha]_D^{23}$ +21.4° (c=0.99, 1N acetic acid)
Rf; 0.67 (n-BuOH:AcOH:$H_2O$:pyridine = 15:3:10:12)

Example 32: $H_2NC(NH)$-Tyr-D-IEPR-Phe-MeβAla-OH • diacetate

$H_2NC(NH)$-Tyr-D-DAPR-Phe-MeβAla-OH • diacetate obtained in Example 11 (1.33 g, 2.00 mmol) was dissolved in dimethylformamide (10 ml), and ethyl acetimidate hydrochloride (494 mg, 4.00 mmol) and triethylamine (0.98 ml, 7.0 mmol) were dissolved in the solution. This solution was stirred at room temperature for 4 hours. The reaction mixture was added with diethyl ether, and the supernatant was removed by decantation. The resulting oily product was charged on an ODS chromatography column (Fuji Silysia DM 1020T, 50g) and eluted stepwise with a gradient of 1-10% acetonitrile/0.1N acetic acid solution. Fractions containing the desired product were collected and lyophilized to obtain 855 mg of the title compound as white powder.

FAB MS m/z : 583 ($M+H^+$)
$[\alpha]_D^{23}$ +21.9° (c=1.00, 1N acetic acid)
Rf; 0.51 (n-BuOH:AcOH:$H_2O$:pyridine = 15:3:10:12)

Example 33: $H_3C$-C(NH)-Tyr-D-AGPR-Phe-MeβAla-OH • diacetate

(1) Boc-Tyr-D-AGPR-Phe-MeβAla-OH

Boc-Tyr(Bzl)-D-DAPR(Z)-Phe-MeβAla-OBzl (1.00 g, 1.08 mmol) was dissolved in acetic acid (10 ml), and added with 0.4 g of 5% Pd-C (water content: 50%) as catalyst, and catalytic reduction was carried out for 4.5 hours to remove the protective group. After the catalyst was removed by filtration, the solvent was concentrated under reduced pressure. The resulting residue was dissolved in dimethylformamide (9 ml), and then 1H-pyrazole-1-carboxamidine • hydrochloride (341 mg, 2.33 mmol) and triethylamine (0.56 ml, 4.0 mmol) were dissolved in the solution. This solution was stirred at room temperature for 48 hours. The reaction mixture was added with diethyl ether, and the supernatant was removed by decantation to give 609 mg of an oily product.

(2) $H_3C$-C(NH)-Tyr-D-AGPR-Phe-MeβAla-OH • diacetate

Boc-Tyr-D-AGPR-Phe-MeβAla-OH obtained in the above (1) (609 mg, 0.87 mmol) was dissolved in 4N HCl/dioxane (10 ml), and stirred at room temperature for 30 minutes. The reaction mixture was added with diethyl ether, and the solvent was removed by decantation. The resulting oily product was dissolved in dimethylformamide (10 ml), and then ethyl acetimidate hydrochloride (215 mg, 1.74 mmol) and triethylamine (0.42 ml, 3.0 mmol) were dissolved in the solution. This solution was stirred at room temperature for 1.5 hours. The reaction mixture was concentrated under reduced pressure, and the resulting oily product was charged on an ODS chromatography column (Fuji Silysia DM 1020T, 100g) and eluted stepwise with a gradient of 1-9% acetonitrile/0.1N acetic acid solution. Fractions containing the desired product were collected and lyophilized to obtain 120 mg of the title compound as white powder.

FAB MS m/z : 583($M+H^+$)
$[\alpha]_D^{23}$ +23.7° (c=1.00, 1N acetic acid)
Rf; 0.56 (n-BuOH:AcOH:$H_2O$:pyridine = 15:3:10:12)

Example 34: $H_3NC(NH)$-Tyr-D-Cit-Phe-MeβAla-OH

(1) N-BZA-Tyr(Bzl)-D-Cit-Phe-MeβAla-OBzl

Boc-Tyr(Bzl)-D-Cit-Phe-MeβAla-OBzl (0.77 g, 0.90 mmol) was dissolved in 4N HCl/dioxane (10 ml) and stirred at room temperature for 30 minutes. The reaction mixture was added with diethyl ether, and the precipitated crystals were collected by filtration. These crystals were dissolved in DMF (10 ml), and 1-(N,N'-bis(benzyloxycarbonyl)amidino)pyrazole (323 mg, 0.86 mmol) and triethylamine (0.15 ml, 1.08 mmol) were dissolved in the solution. The solution was stirred at room temperature for 20 hours. The reaction mixture was added with ethyl acetate (50 ml) and washed with 1N HCl, saturated aqueous sodium hydrogencarbonate and saturated brine. After dried over anhydrous magnesium sulfate, the solvent was concentrated under reduced pressure, and the resulting oily product was purified by silica gel column chromatography (eluted with chloroform:methanol = 30:1) to give 0.76 g of the desired compound as a colorless oily product.

(2) $H_2NC(NH)$-Tyr(Bzl)-D-Cit-Phe-MeβAla-OH

The protected peptide obtained in the above (1) was dissolved in acetic acid (10 ml) and added with 0.5 g of 5% Pd-C (aqueous content: 50%) as catalyst, and then catalytic reduction was carried out for 3.5 hours to remove the protective group. After the catalyst was removed by filtration, the solvent was concentrated under reduced pressure, and the resulting residue was dissolved in 0.1N acetic acid. The solution was lyophilized to obtain 460 mg of the title compound as white powder.

FAB MS m/z : 613 $(M+H^+)$
$[\alpha]_D^{23}$ +22.7° (c=1.19, 1N acetic acid)
Rf; 0.56 (n-BuOH:AcOH:$H_2O$:pyridine = 15:3:10:12)

Example 35: $H_3C$-C(NH)-Tyr-D-Cit-Phe-MeβAla-OH

(1) H-Tyr-D-Cit-Phe-MeβAla-OH

Boc-Tyr(Bzl)-D-Cit-Phe-MeβAla-OBzl (8.75 g, 10.3 mmol) was dissolved in 4N HCl/ethyl acetate (50 ml), and stirred at room temperature for 30 minutes. The reaction mixture was concentrated under reduced pressure, and added with diethyl ether, and the precipitated crystals were collected by filtration. These crystals were dissolved in acetic acid (100 ml) and added with 3.0 g of 5% Pd-C (water content: 50%) as catalyst, and then catalytic reduction was carried out for 3.5 hours to remove the protective group. After the catalyst was removed by filtration, the solvent was concentrated under reduced pressure, and the residue was dissolved in 0.1N acetic acid. This solution was lyophilized to give white powder.

(2) $H_3C$-C(NH)-Tyr-D-Cit-Phe-MeβAla-OH

H-Tyr-D-Cit-Phe-MeβAla-OH obtained in the above (1) was dissolved in dimethylformamide (30 ml), and then ethyl acetimidate hydrochloride (1.66 g, 13.4 mmol) and triethylamine (4.30 ml, 30.8 mmol) were dissolved in the solution. This solution was stirred at room temperature for 3 hours, further added with ethyl acetimidate hydrochloride (618 mg, 5.00 mmol) and triethylamine (0.70 ml, 5.00 mmol), and then stirred for additional 2 hours. The reaction mixture was concentrated under reduced pressure, and the resulting residue was charged on an ODS chromatography column (Fuji Silysia DM 1020T, 500 g) and eluted stepwise with a gradient of 4-10% acetonitrile/0.1N acetic acid solution. Fractions containing the desired product were collected and lyophilized to obtain 4.96 g of the title compound as white powder.

FAB MS m/z : 612$(M+H^+)$
$[\alpha]_D^{23}$ +21.7° (c=1.09, 1N acetic acid)
Rf; 0.60 (n-BuOH:AcOH:$H_2O$:pyridine = 15:3:10:12)

Example 36: $H_3C$-C(NH)-Tyr-D-Gln-Phe-MeβAla-OH

Using Boc-Tyr(Bzl)-D-Gln-Phe-MeβAla-OBzl (1.15 g, 2.00 mmol), ethyl acetimidate hydrochloride (494 mg, 4.00 mmol), and triethylamine (0.84 ml, 6.00 mmol), reactions were carried out as in Example 35. The resulting crude product was charged on an ODS chromatography column (Fuji Silysia DM 1020T, 50 g) and eluted stepwise with a gradient of 10-20% methanol/0.1N acetic acid solution. Fractions containing the desired product were collected and lyophilized to obtain 527 mg of the title compound as white powder.

FAB MS m/z : 583$(M+H^+)$
$[\alpha]_D^{23}$ +24.5° (c=1.01, 1N acetic acid)
Rf; 0.59 (n-BuOH:AcOH:$H_2O$:pyridine = 15:3:10:12)

Example 37: $H_3C$-C(NH)-Tyr-D-Orn(Tfa)-Phe-MeβAla-OH

Using Boc-Tyr(Bzl)-D-Orn(Tfa)-Phe-MeβAla-OBzl (1.00 g, 1.11 mmol), ethyl acetimidate hydrochloride (247 mg, 2.00 mmol), and triethylamine (0.42 ml, 3.00 mmol), reactions were carried out as in Example 35. The resulting crude product was charged on an ODS chromatography column (Fuji Silysia DM 1020T, 50 g) and eluted stepwise with a gradient of 20-30% methanol/0.1N acetic acid solution. Fractions containing the desired product were collected and lyophilized to obtain 115 mg of the title compound as white powder.

FAB MS m/z : 665($M+H^+$)
$[\alpha]_D^{23}$ +24.6° (c=1.01, 1N acetic acid)
Rf ; 0.70 (n-BuOH:AcOH:$H_2O$:pyridine = 15:3:10:12)

Example 38: $H_3C$-C(NH)-Tyr-D-Orn(Ac)-Phe-MeβAla-OH

Using Boc-Tyr(Bzl)-D-Orn(Ac)-Phe-MeβAla-OBzl (2.10 g, 2.47 mmol), ethyl acetimidate hydrochloride (629 mg, 4.94 mmol), and triethylamine (2.06 ml, 14.8 mmol), reactions were carried out as in Example 35. The resulting crude product was charged on an ODS chromatography column (Fuji Silysia DM 1020T, 100g) and eluted stepwise with a gradient of 3-14% acetonitrile/0.1N acetic acid solution. Fractions containing the desired product were collected and lyophilized to obtain 620 mg of the title compound as white powder.

FAB MS m/z : 611($M+H^+$)
$[\alpha]_D^{23}$ +24.6° (c=1.01, 1N acetic acid)
Rf ; 0.59 (n-BuOH:AcOH:$H_2O$:pyridine = 15:3:10:12)

Test Examples

The analgesic activities of the peptide derivatives of the present invention were evaluated by pressure stimulation method. Mice were subjected to pressure stimulation at the bases of their tails at a rate of 10 mmHg/second. Pressure values where the mice showed behaviors such as writhing and biting at the stimulated site were measured and were used as pain reaction thresholds. For the experiments, mice that were preliminarily determined to respond to a pressure of 40-50 mmHg were used. The maximum stimulating pressure was 100 mmHg. Analgesic activity was calculated as percent of maximum possible effect (% of MPE) according to the following equation:

$$\%\ \text{of MPE} = \frac{Pt - Po}{Pc - Po} \times 100$$

wherein Po is the pain reaction threshold before the administration of a drug; Pt is the pain reaction threshold "t" minutes after the administration of the drug; and Pc is the maximum stimulating pressure. The results of analgesic activity obtained from subcutaneous administration (skin in the backs) and oral administration are shown in Table 1.

Table 1

| Peptide Derivative Example No. | MPE(%) | |
|---|---|---|
| | 1 mg/kg s.c. (%) | 10 mg/kg p.o.(%) |
| Example 2 | 93.47 | 22.78 |
| Example 4 | 70.85 | 18.11 |
| Example 7 | 43.29 | 21.34 |
| Example 9 | 22.82 | 9.85 |
| Example 10 | 42.78 | 10.62 |
| Example 12 | 58.92 | 46.82 |
| Example 16 | 94.45 | 66.3 |

Table 1 (continued)

| Peptide Derivative Example No. | MPE(%) | |
|---|---|---|
| | 1 mg/kg s.c. (%) | 10 mg/kg p.o.(%) |
| Example 17 | 100 | 88.34 |
| Example 19 | 87.11 | 82.29 |
| Example 25 | 22.55 | 10.96 |
| Example 26 | 32.83 | 14.44 |
| Example 27 | 52.14 | 33.15 |
| Example 31 | 100 | 96.18 |
| Example 32 | 66.61 | 52.72 |
| Example 35 | 98.8 | 82.06 |

The peptide derivatives of the present invention are useful since they can be used for the treatment of cancerous pain and the like.

**Claims**

1. A compound represented by the following formula or a salt thereof:

$$HN=C(R^1)\text{-}X\text{-}Y\text{-}NH\text{-}CH(R^2)\text{-}CO\text{-}Q$$

wherein:

$R^1$ represents an amino group which may optionally be substituted or a $C_{1-6}$ alkyl group;
$R^2$ represents an aryl-substituted $C_{1-6}$ alkyl group wherein the aryl group may have one or more substituents, or a $C_{3-6}$ cycloalkyl-substituted $C_{1-6}$ alkyl group wherein the cycloalkyl group may have one or more substituents;
X represents a proline residue which may optionally be substituted, or a group represented by the following formula:

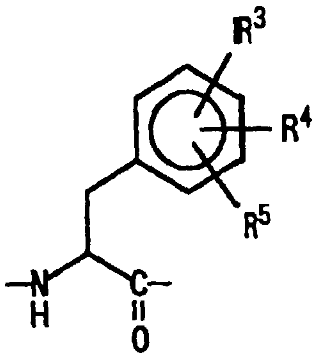

wherein $R^3$, $R^4$ and $R^5$ independently represent hydrogen atom, hydroxyl group, a $C_{1-6}$ alkyl group, a $C_{1-6}$ alkoxyl group, a halogen atom, or $-OSO_3H$;
Y represents a proline residue or a group represented by the following formula:

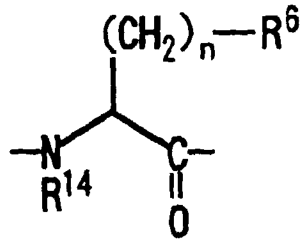

wherein:

$R^6$ represents hydrogen atom, an amino group which may optionally be substituted, a guanidino group which may optionally be substituted, a mercapto group which may optionally be substituted, a phenyl group which may optionally be substituted, a ureido group which may optionally be substituted, a carbamoyl group which may optionally be substituted, a thioureido group which may optionally be substituted, a $C_{1-6}$ alkylsulphinyl group, a $C_{1-6}$ alkylsulfonyl group, a $C_{1-6}$ acyloxy group, carboxyl group, or a $C_{1-6}$ alkoxycarbonyl group; n is an integer of from 1 to 4; and $R^{14}$ represents hydrogen atom or an $C_{1-6}$ alkyl group;

Q represents:

-$OR^7$ wherein $R^7$ represents hydrogen atom or a $C_{1-6}$ alkyl group;

-$N(R^8)(R^9)$ wherein $R^8$ represents hydrogen atom or a $C_{1-6}$ alkyl group, and $R^9$ represents a $C_{1-6}$ hydroxyalkyl group or a sulfonic acid-substituted $C_{1-6}$ alkyl group, or $R^8$ and $R^9$ may combine to form a 5- or 6-membered nitrogen-containing saturated heterocyclic group together with the nitrogen atom to which $R^8$ and $R^9$ bind; or

-$NR^{10}$-$C(R^{11})(R^{12})(R^{13})$ wherein $R^{10}$ represents hydrogen atom, a $C_{1-6}$ alkyl group, or an aryl-substituted $C_{1-6}$ alkyl; $R^{11}$ represents hydrogen atom, carboxyl group, a $C_{1-6}$ alkoxycarbonyl group, a substituted or unsubstituted carbamoyl group, a carboxy-$C_{1-6}$ alkyl group, a substituted or unsubstituted carbamoyl-$C_{1-6}$ alkyl group, or a $C_{1-6}$ alkoxycarbonyl-$C_{1-6}$ alkyl group; $R^{12}$ represents hydrogen atom, a $C_{1-6}$ alkyl group, an amino-$C_{1-6}$ alkyl group, an amidino-$C_{1-6}$ alkyl group, a guanidino-$C_{1-6}$ alkyl group, a hydroxy-$C_{1-6}$ alkyl group, a carboxy-$C_{1-6}$ alkyl group, or a substituted or unsubstituted carbamoyl-$C_{1-6}$ alkyl group, or $R^{10}$ and $R^{12}$ may combine to form a 5- or 6-membered nitrogen-containing saturated heterocyclic group substituted with a carboxyl group together with the nitrogen atom to which $R^{10}$ binds; and $R^{13}$ represents hydrogen atom or a $C_{1-6}$ alkyl group,

provided that a combination wherein $R^2$ is unsubstituted benzyl group, X is L-tyrosine residue, and Y is L- or D-arginine residue is excluded.

2. The compound or the salt according to claim 1, wherein $R^2$ is p-hydroxybenzyl group, 2-phenyl-1-ethyl group, or cyclohexylmethyl group.

3. The compound or the salt according to claim 1 or 2, wherein X is 4-hydroxyproline residue.

4. The compound or the salt according to claim 1 or 2, wherein X is L-tyrosine residue.

5. The compound or the salt according to claims 1 or 2, wherein $R^3$, $R^4$, and $R^5$ represent hydrogen atoms.

6. The compound or the salt according to claim 1 or 2, wherein $R^3$ represents 4-hydroxyl group, $R^4$ represents 2-methyl group, and $R^5$ represents 6-methyl group.

7. The compound or the salt according to claim 1 or 2, wherein $R^3$ represents 4-methoxy group, and $R^4$ and $R^5$ represent hydrogen atoms.

8. The compound or the salt according to claim 1 or 2, wherein $R^3$ represents 3-hydroxyl group, $R^4$ represents 4-hydroxyl group, and $R^5$ represents hydrogen atom.

9. The compound or the salt according to claim 1 or 2, wherein $R^3$ represents - $OSO_3H$, and $R^4$ and $R^5$ represent hydrogen atoms.

**10.** The compound or the salt according to any one of claims 1 through 9, wherein Y is selected from the group consisting of D-alanine residue, D-glutamate residue, D-norvaline residue, D-proline residue, and N-methyl-D-arginine residue.

**11.** The compound or the salt according to any one of claims 1 through 9, wherein Y is selected from the group consisting of citrulline residue, D-methionine-(RS)-sulfoxide residue, D-methionine-(S)-sulfoxide residue, and D-methionine-(R)-sulfoxide residue.

**12.** The compound or the salt according to any one of claims 1 through 11, wherein $R^6$ represents unsubstituted amino group, a mono-$C_{1-6}$ alkylamino group or a mono(1-imino-$C_{1-6}$ alkyl)amino group.

**13.** The compound or the salt according to any one of claims 1 through 11, wherein $R^6$ represents unsubstituted guanidino group or a mono-$C_{1-6}$ alkyl-guanidino group.

**14.** The compound or the salt according to any one of claims 1 through 11, wherein $R^6$ represents unsubstituted ureido group or an N'-mono-$C_{1-6}$ alkyl-ureido group.

**15.** The compound or the salt according to any one of claims 1 through 11, wherein $R^6$ represents unsubstituted carbamoyl group or a mono-$C_{1-6}$ alkyl-carbamoyl group.

**16.** The compound or the salt according to any one of claims 1 through 11, wherein $R^6$ represents unsubstituted thioureido group or an N'-mono-$C_{1-6}$ alkyl-thioureido group.

**17.** The compound or the salt according to any one of claims 1 through 11, wherein $R^6$ represents p-aminophenyl group or p-guanidinophenyl group.

**18.** The compound or the salt according to any one of claims 1 through 17, wherein Q represents $-N(CH_3)-CH_2-CH_2-COOH$.

**19.** The compound or the salt according to any one of claims 1 through 18, wherein $R^1$ represents unsubstituted amino group.

**20.** The compound or the salt according to any one of claims 1 through 19, wherein the absolute configuration of the carbon atom bound to $R^2$ is S-configuration.

**21.** The compound or the salt according to any one of claim 1 through 20, wherein $R^{14}$ represents hydrogen atom.

**22.** The compound or the salt according to claim 13, wherein $R^{14}$ represents a $C_{1-6}$ alkyl group.

**23.** The salt of the compound according to any one of claims 1 through 22, which is in the form of hydrochloride or acetate.

**24.** A medicament comprising the compound or the salt according to any one of claims 1 through 23.

**25.** An analgesic pharmaceutical composition comprising as an active ingredient the compound or the salt according to any one of claims 1 through 23.

**26.** Use of the compound or the salt according to any one of claims 1 through 23 for the manufacture of the analgesic pharmaceutical composition according to claim 25.

**27.** A method for preventive and/or therapeutic treatment of pain which comprises a step of administering to a mammal an effective amount of the compound or the salt according to any one of claims 1 through 23.

**INTERNATIONAL SEARCH REPORT**

International application No.
PCT/JP96/02573

**A. CLASSIFICATION OF SUBJECT MATTER**

Int. $Cl^6$ C07K5/087, 5/107, 5/023, A61K38/06, 38/07

According to International Patent Classification (IPC) or to both national classification and IPC

**B. FIELDS SEARCHED**

Minimum documentation searched (classification system followed by classification symbols)

Int. $Cl^6$ C07K5/02-033, 5/087, 5/107, A61K38/06, 38/07

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| PA | WO, 95/24421, A (Daiichi Seiyaku Co., Ltd.), September 14, 1995 (14. 09. 95) & JP, 7-300496, A | 1 - 26 |
| A | JP, 61-103898, A (Farmitalia Carlo Erba S.p.A.), May 22, 1986 (22. 05. 86) & GB, 2166139, A & BE, 903493, A & DE, 3537405, A | 1 - 26 |
| X | SALVADORI, S. et al., Opioid peptides. Structure-activity relationships in dermorphin tetrapeptides I. Farmaco, Ed. Sci. 1982, Vol. 37, No. 8, p.514-p.518 (particularly, refer to page 516, table I Comp. (IX), (XI), (XII)) | 1, 4, 10, 19-21, 23-26 |
| X | SALVADORI, S. et al., Synthesis and pharmacological activity of dermorphin tetrapeptide-analogs. Eur. J. Med. Chem. - Chim. Ther. 1983, Vol. 18, No. 6, p.489-p.493 (particularly, refer to page | 1, 4, 10, 19-21, 24-26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| November 21, 1996 (21. 11. 96) | December 3, 1996 (03. 12. 96) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP96/02573

C (Continuation). DOCUMENTS CONSIDERED TO BE RELEVANT

| Category* | Citation of document, with indication, where appropriate, of the relevant passages | Relevant to claim No. |
|---|---|---|
| | 490, table II; page 491, Table III; page 492, right column, compounds 20 to 23) | |
| X | SALVADORI, S. et al., Synthesis and opioid activity of (Sar4)dermorphintetrapeptide analogs. Hoppe-Seyler's Z. Physiol. Chem. 1984, Vol. 365, No. 10, p.1199-p.1206 (particularly, refer to page 1201, table 1; page 1205, left column, compounds VI) | 1, 4, 10, 19, 20, 22-26 |
| X | MARASTONI, M. et al., Synthesis and activity profiles of new dermorphin-(1-4) peptide analogs. J. Med. Chem. 1987, Vol. 30, No. 9, p.1538-p.1542 (particularly, refer to page 1538 Scheme I; pages 1539 to 1540, table II. to IV.; page 1542, left column, compounds III, IV, VII, XI) | 1, 4, 11, 19-26 |
| X | CASTIGLIONE-MORELLI, M.A. et al., A 500-MHz proton nuclear magnetic resonance study of μ opioid peptides in a simulated receptor environment. J. Med. Chem. 1987, Vol. 30, No. 11, p.2067-p.2073 (particularly, refer to page 2072, Experimental Section) | 1, 5, 10, 19-21, 23-26 |

Form PCT/ISA/210 (continuation of second sheet) (July 1992)

**INTERNATIONAL SEARCH REPORT**

International application No.
PCT/JP96/02573

**Box I Observations where certain claims were found unsearchable (Continuation of item 1 of first sheet)**

This international search report has not been established in respect of certain claims under Article 17(2)(a) for the following reasons:

1. [X] Claims Nos.: 27
because they relate to subject matter not required to be searched by this Authority, namely:
Claim 27 pertains to methods for treatment of the human or animal body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (1)) (July 1992)